# EUROPEAN PATENT APPLICATION

(11) **EP 4 578 860 A1**
(43) Date of publication of application: **02.07.2025**
(21) Application number: 23857449.5
(22) Date of filing: 25.08.2023
(51) Int. Cl.: C07D 491/113, C07D 491/20, C07D 487/10, C07D 487/20, C07D 498/10

(54) **CYCLOALKYNE DERIVATIVE**

(30) Priority: 26.08.2022 JP 2022135334
(71) Applicant: PeptiDream Inc., Kawasaki-shi, Kanagawa 210-0821 (JP)
(72) Inventor: YAMAKOSHI, Shuhei, Kawasaki-shi, Kanagawa 210-0821 (JP); MATSUMOTO, Masatoshi, Kawasaki-shi, Kanagawa 210-0821 (JP); KINEBUCHI, Masahiko, Kawasaki-shi, Kanagawa 210-0821 (JP); MIZUKOSHI, Yoshihide, Kawasaki-shi, Kanagawa 210-0821 (JP); UEDA, Kyosuke, Kawasaki-shi, Kanagawa 210-0821 (JP); MASUYA, Keiichi, Kawasaki-shi, Kanagawa 210-0821 (JP); AOKI, Takanori, Kawasaki-shi, Kanagawa 210-0821 (JP)
(74) Representative: Mewburn Ellis LLP
(86) International application number: PCT/JP2023/030810
(87) International publication number: WO 2024/043341

(57) **Abstract**

The present invention provides a novel strain-promoted azide alkyne cycloaddition (SPAAC). The present invention provides a novel cycloalkyne derivative having a spiro ring, as a cycloalkyne derivative that can be used for SPAAC. The present invention also provides a method for producing a conjugate of functional molecules by using the cycloalkyne derivative.

## Description

### TECHNICAL FIELD

The present disclosure relates to a cycloalkyne derivative and use thereof.

### BACKGROUND ART

Click chemistry is a technique for synthesizing a new molecule through a carbon-heteroatom bond-forming reaction between compounds having relatively simple structures with a high reactivity and selectivity (Non Patent Literature 1). A 1,3-dipolar cycloaddition between an azide and an alkyne (azide alkyne cycloaddition, AAC), which is a representative click chemistry reaction, allows for the formation of a stable 1,2,3-triazole ring. Thus, this reaction is used as a technique for tightly linking substrates together in the synthesis of a medicament and/or a functional material. In recent years, a strain-promoted azide alkyne cycloaddition (SPAAC) has been developed and has been gaining attention; this reaction activates an alkyne molecule without using a metal catalyst, enabling it to react with an azide compound (Patent Literatures 1 to 3 and Non Patent Literatures 2 to 11). Various cycloalkyne derivatives have been developed for use in SPAAC.

### CITATION LIST

### PATENT LITERATURE

PTL 1: International Publication No. WO 2006/050262
PTL 2: Japanese Patent Laid-Open No. 2018-039773
PTL 3: Japanese Patent Laid-Open No. 2020-026476

### NON PATENT LITERATURE

NPL 1: Kolb, H. C. et al., Angew. Chem. Int. Ed. 2001, 40, 2004.
NPL 2: Dones, J. M., et al., J.Am. Chem. Soc. 2021_143_9489
NPL 3: Danilkina, N. A. et al., J.Am. Chem. Soc.2021_143_16519
NPL 4: Ni, R. et al., Angew. Chem. Int. Ed. 2015_54_1190
NPL 5: Kuzmin, A. et al., Bioconjugate_Chem. 2010_21_2076
NPL 6: Masuda, R. et al., Chem_Asian_J. 2020_15_742
NPL 7: Debets, M. F. et al., Chem. Commun. 2010_46_97
NPL 8: Jewett, J. C. et al., Chem. Soc. Rev. 2010_39_1272
NPL 9: Agard, N. J. et al., J.Am. Chem. Soc. 2004_126_15046
NPL 10: Kawasaki, Y. et al., Chemistry Letters, 2019, 48, 495
NPL 11: Harris, T. et al., Mendeleev Commun. 2019, 29, 237.
NPL 12: Teobald, B. J., Tetrahedron 2002, 58, 4133.

### SUMMARY OF INVENTION

### TECHNICAL PROBLEM

The substrate of SPAAC is not only widely used as a substrate for click chemistry, but also expected to be used to conjugate a carrier peptide with a payload in a peptide-drug conjugate (PDC). Thus, there is a need for a new SPAAC substrate.

### SOLUTION TO PROBLEM

The present inventors conducted extensive studies and found a method for preparing a novel cycloalkyne compound having a spiro ring structure. In addition, the present inventors found that the compound possesses a desirable property as a substrate for SPAAC, thereby completing the present invention.

This specification includes the following disclosure of the invention.
[1-1] A compound represented by formula (I): 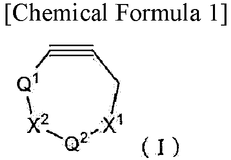 or a salt thereof;
   wherein X¹ and X² are each independently an oxygen atom or -N(Y¹)-,
   Q¹ is either 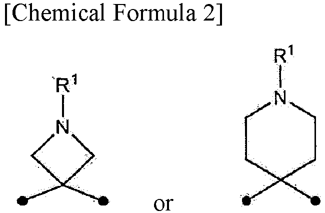 and Q² is selected from C₂₋₄ alkylenes; or
      Q¹ is methylene and Q² is either 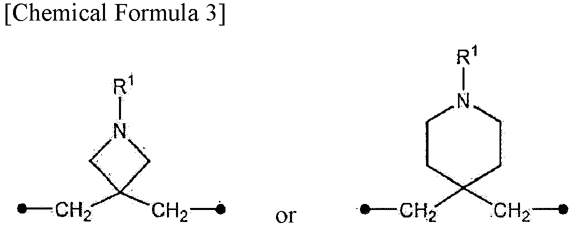
      ; and Y¹ is selected from a hydrogen atom, C₁₋₆ alkanesulfonyl optionally substituted with one or more halogen atoms, and nitrobenzenesulfonyl;
      R¹ is selected from a hydrogen atom, C₁₋₆ alkyl, (C₁₋₆ alkyl)carbonyl, (C₁₋₆ alkoxy)carbonyl, benzyloxycarbonyl, -CO-Q³-X³, -COO-Q³-X³, -CONR²-Q³-X³, -SO₂-Q³-X³, and -SO₂NR²-Q³-X³;
      R² is a hydrogen atom or C₁₋₆ alkyl;
      X³ is -COR⁵, -OL¹, or -NR³R⁴;
      R³ is a hydrogen atom or C₁₋₆ alkyl;
      R⁴ is a hydrogen atom, (C₁₋₆ alkyl)carbonyl, (C₁₋₆ alkoxy)carbonyl, -COX⁴, or benzyloxycarbonyl;
      Q³ is C₁₋₁₀ alkylene or -(CH₂CH₂O)ₙCH₂CH₂-;
      n is an integer of 1 to 10;
      L¹ is a hydrogen atom or -COX⁴;
      R⁵ is hydroxy, X⁴, or C₁₋₆ alkoxy; and
      X⁴ is 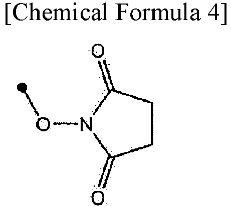
      ; and the triple bond in the compound of formula (I) may be protected by Co₂(CO)₆.
[1-2] The compound according to [1-1] or a salt thereof, wherein R¹ is selected from -CO-CH₂-OH, benzyloxycarbonyl, and tert-butoxycarbonyl.
[1-3] The compound according to [1-1] or [1-2] or a salt thereof, wherein Y¹ is C₁₋₆ alkanesulfonyl, trifluoromethanesulfonyl, and 2-nitrobenzenesulfonyl.
[1-4] The compound according to [1-3] or a salt thereof, wherein Y¹ is methanesulfonyl or tert-butanesulfonyl.
[1-5] The compound according to any of [1-1] to [1-4] or a salt thereof, wherein Q¹ is either 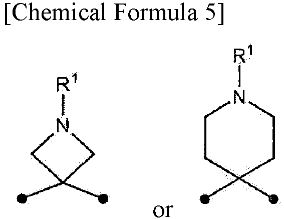
[1-6] The compound according to any of [1-1] to [1-4] or a salt thereof, wherein Q² is either 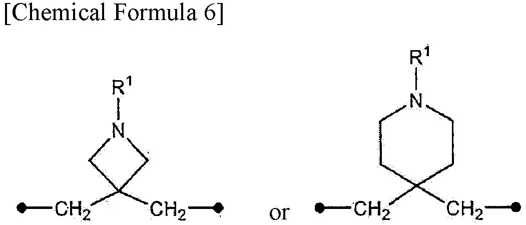
[1-7] The compound according to any of [1-1] to [1-4] or a salt thereof, wherein
   X² is an oxygen atom,
   Q¹ is 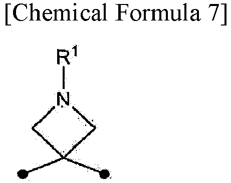
   ; and R¹ is benzyloxycarbonyl.
[1-8] The compound according to any of [1-1] to [1-4] or a salt thereof,
   wherein the compound is an 8- to 9-membered heterocycle; and
   wherein
   Q¹ is 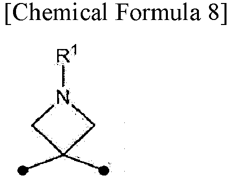
   ; or Q² is 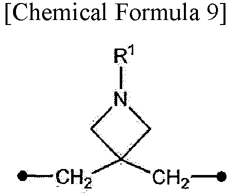
   ; and R¹ is benzyloxycarbonyl.
[1-9] The compound according to [1-1] or a salt thereof,
   wherein the compound is selected from: 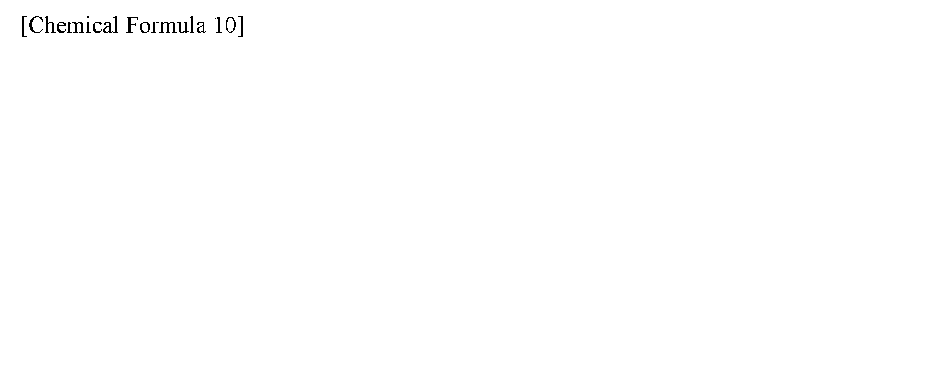 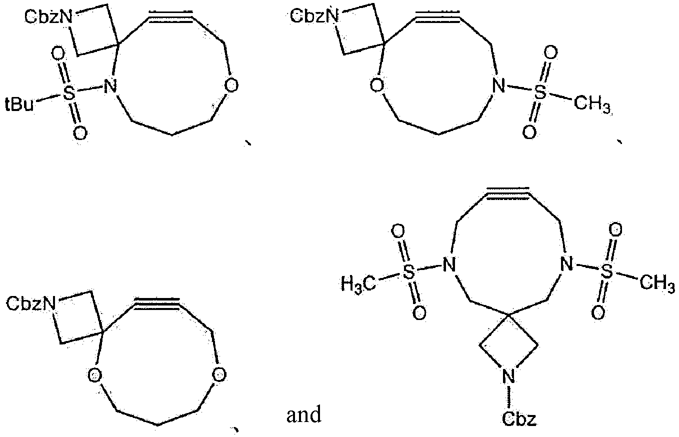
[1-10] A method for producing a conjugate of functional molecules by using the compound according to any of [1-1] to [1-9] or a salt thereof, the method including:
   reacting a first functional molecule having a group represented by formula (II): 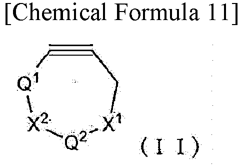
   wherein X¹ and X² are each independently an oxygen atom or -N(Y¹)-,
   Q¹ is either 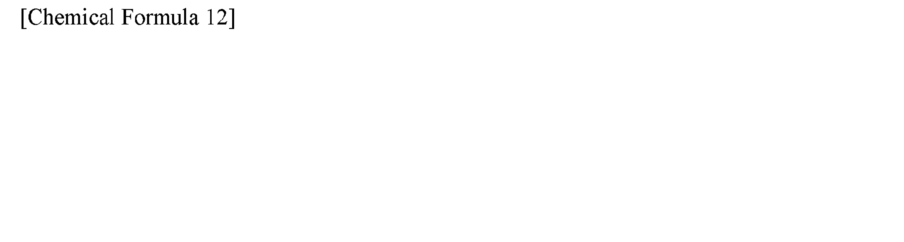 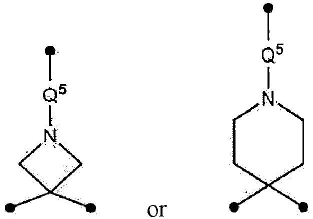 and Q² is selected from C₂₋₄ alkylenes; or
      Q¹ is methylene and Q² is either 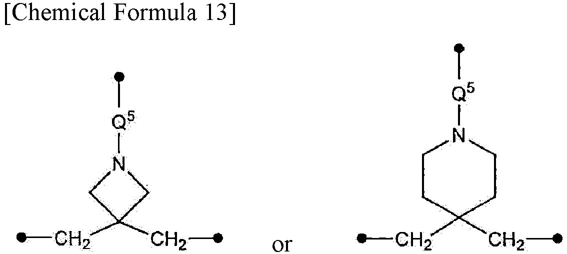
   ; Q⁵ is a linker or a single bond attached to the first functional molecule with a second functional molecule having a group represented by formula (III):

      -Q⁶-N₃ (III)

      wherein Q⁶ is a linker or a single bond attached to the second functional molecule to obtain a conjugate including the first functional molecule and the second functional molecule linked by a linker, represented by formula IVa or formula IVb: 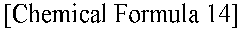 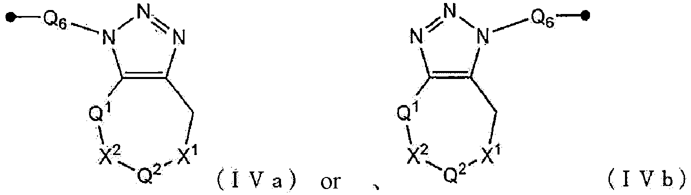
[1-11] The method according to [1-10], wherein the first functional molecule and the second functional molecule are each independently a pharmacologically active compound, a label compound, a peptide, a protein, a nucleic acid, or a molecule used for a drug delivery system.
[1-12] The method according to [1-11], wherein either the first functional molecule or the second functional molecule is a pharmacologically active compound, a label compound, a peptide, a protein, or a nucleic acid that has a specific binding activity to a target.
[1-13] A conjugate including a first functional molecule and a second functional molecule linked by a linker, represented by formula (IVa) or formula (IVb): 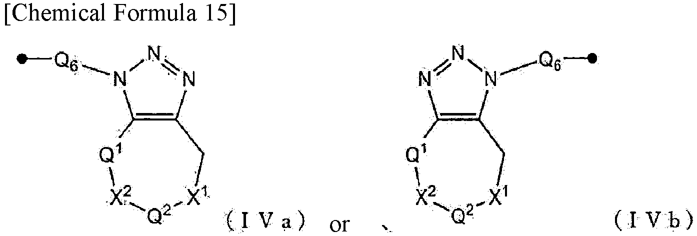 wherein X¹, X², Q¹, Q², and Q⁶ are as defined in [1-10].
[1-14] The conjugate according to [1-13], wherein the first functional molecule and the second functional molecule are each independently a pharmacologically active compound, a label compound, a peptide, a protein, a nucleic acid, or a molecule used for a drug delivery system.
[1-15] The conjugate according to [1-14], wherein either the first functional molecule or the second functional molecule is a pharmacologically active compound, a label compound, a peptide, a protein, or a nucleic acid that has a specific binding activity to a target.
[1-16] A method for producing a compound represented by formula (I): 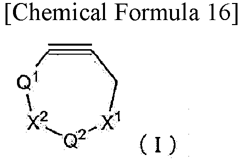 wherein X¹, X², Q¹, and Q² are as defined in [1-1], and the triple bond in the compound of formula (I) may be protected by Co₂(CO)₆;
   the method including:
   treating a compound represented by formula (V): 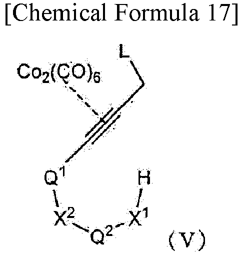
   wherein X¹, X², Q¹, and Q² are as defined in [1-1], with the provision that any hydroxy group and amino group, if present, is protected by a protecting group; and L is a leaving group,
   in the presence of a Lewis acid to obtain a corresponding closed-ring compound.
[1-17] The method according to [1-16], wherein L is -OR¹⁰ or a halogen atom; and R¹⁰ is a hydrogen atom, (C₁₋₆ alkyl) carbonyl, acetyl, tri(C₁₋₆ alkyl)silyl, C₁₋₆ alkyl, benzenesulfonyl, or C₁₋₆ alkanesulfonyl.
[2-1] A compound represented by formula (I): 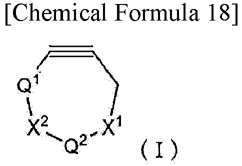 or a salt thereof;
   wherein X¹ and X² are each independently an oxygen atom or -N(Y¹)-;
   Q¹ is either 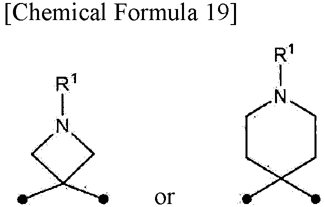 and Q² is selected from C₂₋₄ alkylenes; or
      Q¹ is methylene and Q² is either 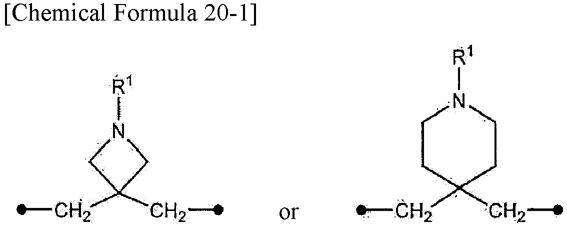
      ; and Y¹ is selected from a hydrogen atom, C₁₋₆ alkanesulfonyl optionally substituted with one or more halogen atoms, and nitrobenzenesulfonyl;
      R¹ is selected from a hydrogen atom, C₁₋₆ alkyl, (C₁₋₆ alkyl)carbonyl, (C₁₋₆ alkoxy)carbonyl, benzyloxycarbonyl, [(9H-fluoren-9-yl)methoxy]carbonyl, -CO-Q³-X³, - COO-Q³-X³, -CONR²-Q³-X³, -SO₂-Q³-X³, and -SO₂NR²-Q³-X³;
      R² is a hydrogen atom or C₁₋₆ alkyl;
      X³ is *-*COR⁵, -OL¹, -NR³R⁴, or 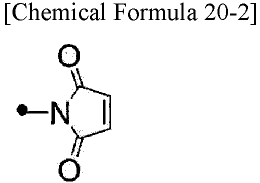
         R³ is a hydrogen atom or C₁₋₆ alkyl;
         R⁴ is a hydrogen atom, (C₁₋₆ alkyl)carbonyl, (C₁₋₆ alkoxy)carbonyl, -COX⁴, or benzyloxycarbonyl;
         Q¹ is C₁₋₁₀ alkylene, cyclo C₃₋₁₀ alkylene, C₂₋₁₀ alkylene in which cyclo C₃₋₁₀ alkylene, -(cyclo C₃₋₁₀ alkylene)-(C₁₋₁₀ alkylene)-, -(C₁₋₁₀ alkylene)-(cyclo C₃₋₁₀ alkylene)-, or - (CH₂CH₂O)ₙCH₂CH₂- is inserted;
         n is an integer of 1 to 10;
         L¹ is a hydrogen atom, -COX⁴, or (C₁₋₆ alkyl)diphenylsilyl;
         R⁵ is hydroxy, X⁴, or C₁₋₆ alkoxy; and
         X⁴ is 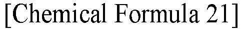 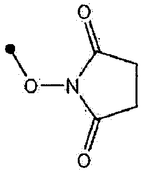
         ; and the triple bond in the compound of formula (I) may be protected by Co₂(CO)₆.
[2-2] The compound according to [2-1] or a salt thereof, wherein Q³ is ethylene or propylene.
[2-3] The compound according to [2-1] or [2-2] or a salt thereof, wherein R¹ is selected from -CO-CH₂-OH, benzyloxycarbonyl, tert-butoxycarbonyl, [(9H-fluoren-9-yl)methoxy]carbonyl.
[2-4] The compound according to any of [2-1] to [2-3] or a salt thereof, wherein Y¹ is C₁₋₆ alkanesulfonyl, trifluoromethanesulfonyl, and 2-nitrobenzenesulfonyl.
[2-5] The compound according to [2-4] or a salt thereof, wherein Y¹ is methanesulfonyl or tert-butanesulfonyl.
[2-6] The compound according to any of [2-1] to [2-5] or a salt thereof, wherein Q¹ is either 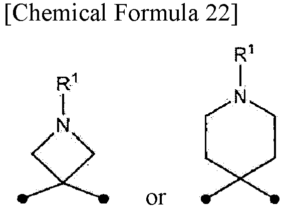
[2-7] The compound according to any of [2-1] to [2-5] or a salt thereof, wherein Q² is either 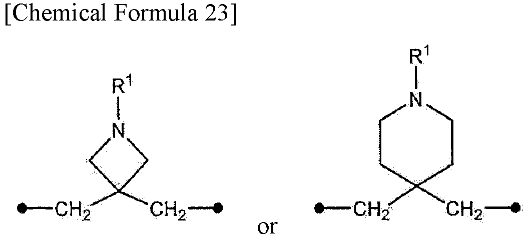
[2-8] The compound according to any of [2-1] to [2-5] or a salt thereof,
   wherein Q¹ is 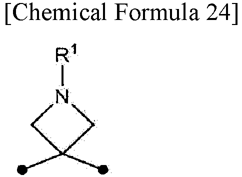 ; and R¹ is benzyloxycarbonyl.
[2-9] The compound according to any of [2-1] to [2-5] or a salt thereof,
   wherein Q¹ is 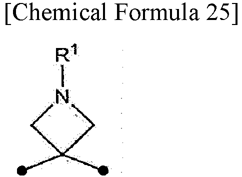 and Q² is selected from C₂₋₃ alkylenes or Q² is 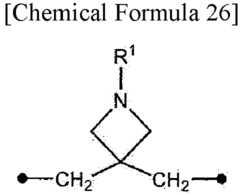 ; and R¹ is benzyloxycarbonyl.
[2-10] The compound according to [2-1] or a salt thereof, wherein the compound is selected from: 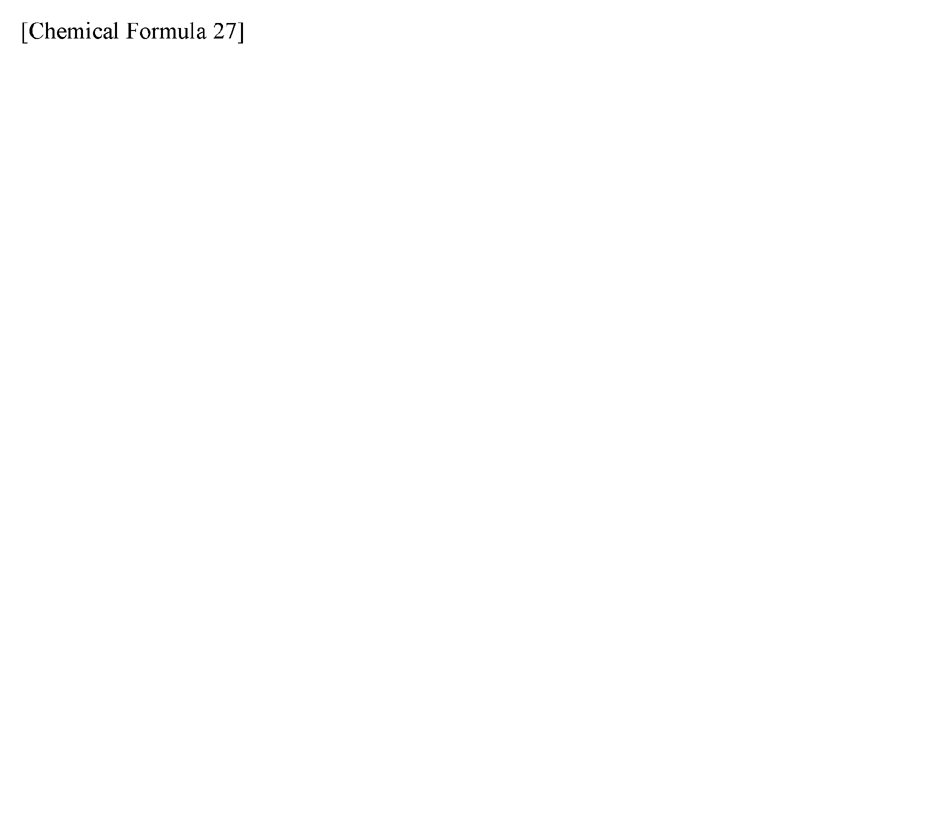 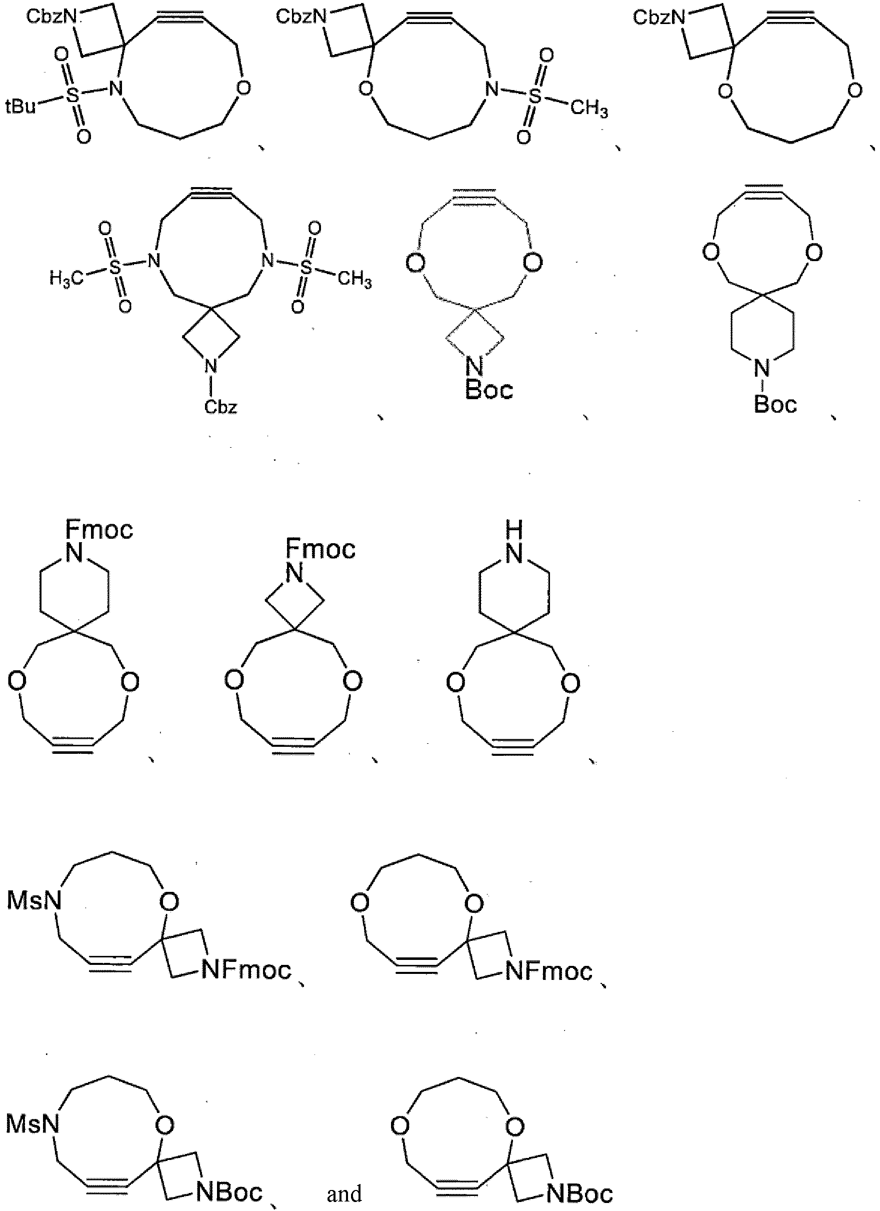
[2-11] A method for producing a conjugate of functional molecules by using the compound according to any of [2-1] to [2-10] or a salt thereof, the method including:
   reacting a first functional molecule having a group represented by formula (II): 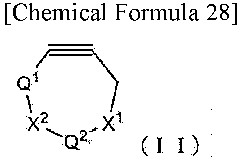
   wherein X¹ and X² are each independently an oxygen atom or -N(Y¹)-;
   Q¹ is either 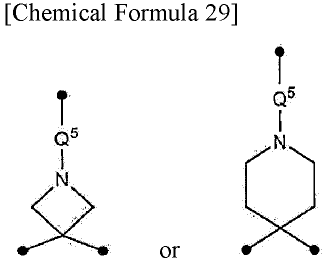 and Q² is selected from C₂₋₄ alkylenes; or
      Q¹ is methylene and Q² is either 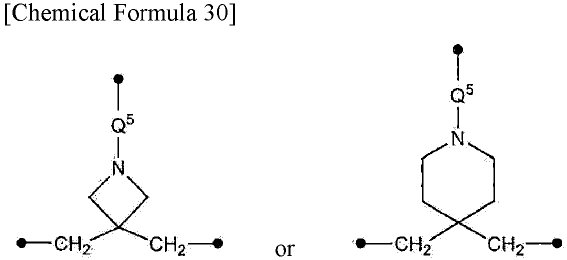 ; Q⁵ is a linker or a single bond attached to the first functional molecule with a second functional molecule having a group represented by formula (III):

      -Q⁶-N₃ (III)

      wherein Q⁶ is a linker or a single bond attached to the second functional molecule to obtain a conjugate including the first functional molecule and the second functional molecule linked by a linker, represented by formula IVa or formula IVb: 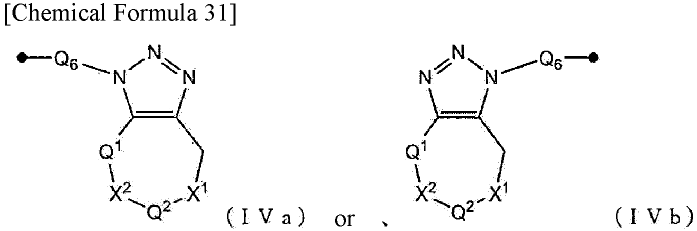
[2-12] The method according to [2-11], wherein the first functional molecule and the second functional molecule are each independently a pharmacologically active compound, a label compound, a peptide, a protein, a nucleic acid, or a molecule used for a drug delivery system.
[2-13] The method according to [2-12], wherein either the first functional molecule or the second functional molecule is a pharmacologically active compound, a label compound, a peptide, a protein, or a nucleic acid that has a specific binding activity to a target.
[2-14] A conjugate including a first functional molecule and a second functional molecule linked by a linker, represented by formula (IVa) or formula (IVb): 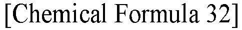 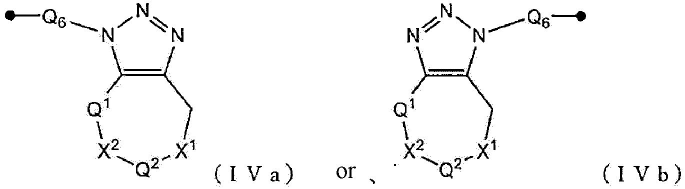 wherein X¹, X², Q¹, Q², and Q⁶ are as defined in [2-11].
[2-15] The conjugate according to [2-14], wherein the first functional molecule and the second functional molecule are each independently a pharmacologically active compound, a label compound, a peptide, a protein, a nucleic acid, or a molecule used for a drug delivery system.
[2-16] The conjugate according to [2-15], wherein either the first functional molecule or the second functional molecule is a pharmacologically active compound, a label compound, a peptide, a protein, or a nucleic acid that has a specific binding activity to a target.
[2-17] A method for producing a compound represented by formula (I): 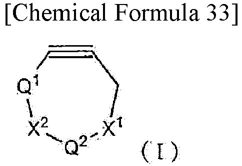 wherein X¹, X², Q¹, and Q² are as defined in [2-1], and the triple bond in the compound of formula (I) may be protected by Co₂(CO)₆;
   the method including:
   treating a compound represented by formula (V): 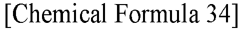 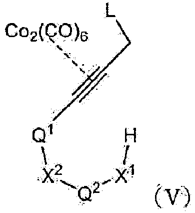
   wherein X¹, X², Q¹, and Q² are as defined in [2-1], with the provision that any hydroxy group and amino group, if present, is protected by a protecting group; and L is a leaving group,
   in the presence of an acid to obtain a corresponding closed-ring compound.
[2-18] The producing method according to [2-17], wherein the acid is a Lewis acid or a Bronsted acid.
[2-19] The method according to [2-17], wherein L is -OR¹⁰ or a halogen atom; and R¹⁰ is a hydrogen atom, (C₁₋₆ alkyl)carbonyl, acetyl, tri(C₁₋₆ alkyl)silyl, C₁₋₆ alkyl, benzenesulfonyl, or C₁₋₆ alkanesulfonyl.

### ADVANTAGEOUS EFFECTS OF INVENTION

The novel cycloalkyne derivative having a spiro ring according to the present invention is highly universal as a cycloalkyne derivative that can be used for SPAAC, and is also useful for conjugation to produce a PDC.

### BRIEF DESCRIPTION OF DRAWINGS

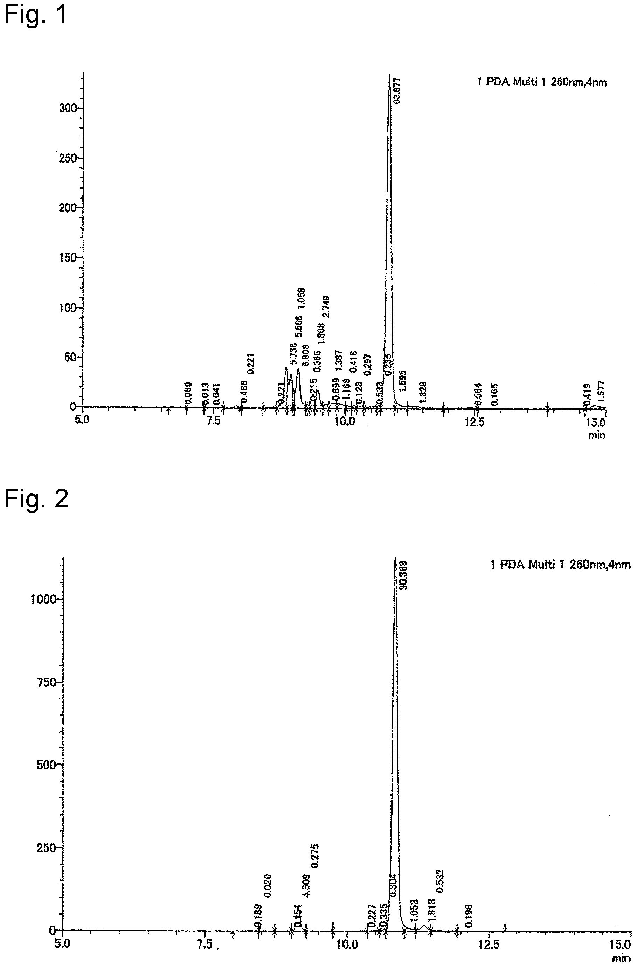

[Fig. 1] Fig. 1 is a graph showing the results of an HPLC analysis, where the synthesis of PN-S01-10 synthesized in [Example 13-1] was analyzed under analysis condition 1.
[Fig. 2] Fig. 2 is a graph showing the results of an HPLC analysis, where the freeze-dried product of PN-S01-10 obtained in [Example 13-4] was analyzed under analysis condition 1.

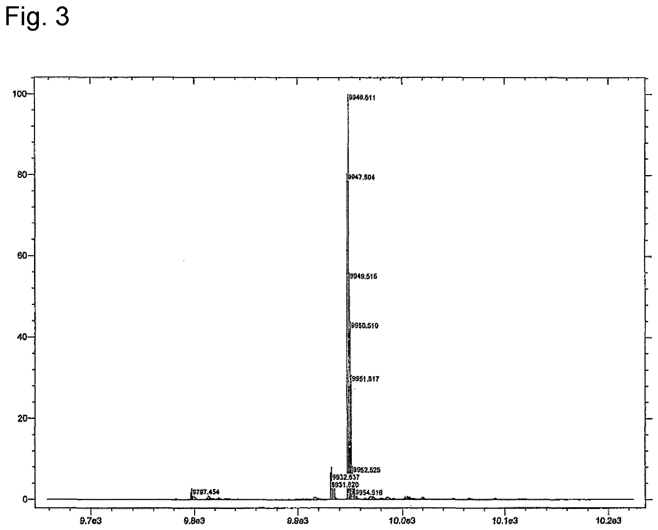

[Fig. 3] Fig. 3 is a graph showing the results of molecular weight measurement, where the freeze-dried product of PN-S01-10 obtained in [Example 13-4] was analyzed under analysis condition 1.
[Fig. 4] Fig. 4 shows the structural formula of a nucleic acid compound (synthesized according to a general synthetic method with reference to US20180010126; hereinafter, this may be referred to as NS-S01-20).

### DESCRIPTION OF EMBODIMENTS

The present invention, in one embodiment, provides a novel cycloalkyne derivative that can be used in SPAAC.

As used herein, the term "halogen atom" refers to a fluorine atom, a chlorine atom, a bromine atom, an iodine atom, or the like. When a halogen atom is used as a substituent for groups such as aryl or heteroaryl, examples of such halogen atoms include a fluorine atom, a chlorine atom, and a bromine atom. In the present specification, when a halogen atom is used as a substituent for an alkyl group or an alkyl-containing group (e.g., alkoxy, alkenyl, alkylthio), examples of such halogen atoms include a fluorine atom. Specific examples of groups having a halogen atom as a substituent include trifluoromethyl, pentafluoroethyl, trifluoromethoxy, pentafluoroethoxy, trifluoromethylthio, and pentafluoroethylthio.

As used herein, the term "C₁₋₆ alkyl" refers to a monovalent group that is derived from a linear or branched saturated aliphatic hydrocarbon having 1 to 6 carbon atoms by removing any single hydrogen atom. Specific examples include methyl, ethyl, n-propyl, isopropyl, n-butyl, isobutyl, sec-butyl, t-butyl, 1-methylpropyl, n-pentyl, isopentyl, 2-methylbutyl, 1,1-dimethylpropyl, 1-ethylpropyl, hexyl, and 4-methylpentyl.

As used herein, the term "C₁₋₆ alkoxy" refers to a C₁₋₆ alkyl-O- group, wherein C₁₋₆ alkyl is as defined above. Specific examples include methoxy, ethoxy, 1-propoxy, 2-propoxy, n-butoxy, i-butoxy, sec-butoxy, t-butoxy, 1-pentyloxy, and 1-hexyloxy.

As used herein, the term "(C₁₋₆ alkyl)carbonyl" refers to a C₁₋₆ alkyl-C(O)- group, wherein C₁₋₆ alkyl is as defined above. Specific examples include methylcarbonyl, ethylcarbonyl, n-propylcarbonyl, i-propylcarbonyl, n-butylcarbonyl, i-butylcarbonyl, sec-butylcarbonyl, t-butylcarbonyl, 1-methylpropylcarbonyl, n-pentylcarbonyl, isopentylcarbonyl, 2-methylbutylcarbonyl, 1,1-dimethylpropylcarbonyl, 1-ethylpropylcarbonyl, hexylcarbonyl, 4-methylpentylcarbonyl, and 2-ethylbutylcarbonyl.

As used herein, the term "(C₁₋₆ alkoxy)carbonyl" refers to a C₁₋₆ alkoxy-C(O)- group, wherein C₁₋₆ alkoxy is as defined above. Specific examples include methoxycarbonyl, ethoxycarbonyl, 1-propoxycarbonyl, 2-propoxycarbonyl, n-butoxycarbonyl, i-butoxycarbonyl, sec-butoxycarbonyl, t-butoxycarbonyl, 1-pentyloxycarbonyl, and 1-hexyloxycarbonyl.

As used herein, the term "tri(C₁₋₆ alkyl)silyl" refers to a (C₁₋₆ alkyl)₃Si- group, wherein C₁₋₆ alkyl is as defined above and the three alkyl groups may be the same as or different from each other. Specific examples include trimethylsilyl, triethylsilyl, tripropylsilyl, tributylsilyl, tripentylsilyl, and trihexylsilyl.

As used herein, the term "(C₁₋₆ alkyl)diphenylsilyl" refers to a (C₁₋₆ alkyl)(C₆H₅)₂Si-group, wherein C₁₋₆ alkyl is as defined above. Specific examples include t-butyldiphenylsilyl.

As used herein, the term "C₁₋₆ alkanesulfonyl" refers to a C₁₋₆ alkyl-SO₂- group, wherein C₁₋₆ alkyl is as defined above. Specific examples include methylsulfonyl, ethylsulfonyl, and n-propylsulfonyl. C₁₋₆ alkanesulfonyl is, for example, methylsulfonyl.

As used herein, the term "C₁₋₁₀ alkylene" is a group having a -(CₙH₂ₙ)- structure, wherein n is any integer selected from 1 to 10. C₁₋₁₀ alkylene may be substituted or unsubstituted and linear or branched unless otherwise stated. C₁₋₁₀ alkylene includes C₁₋₆ alkylene. Specific examples include methylene, ethylene, propylene, butylene, pentylene, hexylene, heptylene, octylene, nonylene, and decylene.

Furthermore, examples of C₂₋₁₀ alkylene in which cyclo C₃₋₁₀ alkylene is inserted include ethylene in which cyclohexylene is inserted, propylene in which cyclohexylene is inserted, butylene in which cyclohexylene is inserted, pentylene in which cyclohexylene is inserted, octylene in which cyclohexylene is inserted, nonylene in which cyclohexylene is inserted, decylene in which cyclohexylene is inserted; ethylene in which cycloheptylene is inserted, propylene in which cycloheptylene is inserted, butyrene in which cycloheptylene is inserted, pentylene in which cycloheptylene is inserted, octylene in which cycloheptylene is inserted, nonylene in which cycloheptylene is inserted, decylene in which cycloheptylene is inserted; ethylene in which cyclooctylene is inserted, propylene in which cyclooctylene is inserted, butyrene in which cyclooctylene is inserted, pentylene in which cyclooctylene is inserted, octylene in which cyclooctylene is inserted, nonylene in which cyclooctylene is inserted, and decylene in which cyclooctylene is inserted.

In the case of -(cyclo C₃₋₁₀ alkylene)-(C₁₋₁₀ alkylene)-, -(C₁₋₁₀ alkylene)- moiety is attached to X³. In the case of -(C₁₋₁₀ alkylene)-(cyclo C₃₋₁₀ alkylene)-, -(cyclo C₃₋₁₀ alkylene)- moiety is attached to X³. Examples of -(C₁₋₁₀ alkylene)-(cyclo C₃₋₁₀ alkylene)-include methylenecyclopropylene, methylenecyclobutylene, methylenecyclopentylene, methylenecyclohexylene, methylenecycloheptylene, methylenecyclooctylene, methylenecyclononylene, methylenecyclodecylene, ethylenecyclopropylene, ethylenecyclobutylene, ethylenecyclopentylene, ethylenecyclohexylene, ethylenecycloheptylene, ethylenecyclooctylene, ethylenecyclononylene, ethylenecyclodecylene, propylenecyclopropylene, propylenecyclobutylene, propylenecyclopentylene, propylenecyclohexylene, propylenecycloheptylene, propylenecyclooctylene, propylenecyclononylene, propylenecyclodecylene, butylenecyclopropylene, butylenecyclobutylene, butylenecyclopentylene, butylenecyclohexylene, butylenecycloheptylene, butylenecyclooctylene, butylenecyclononylene, butylenecyclodecylene, pentylenecyclopropylene, pentylenecyclobutylene, pentylenecyclopentylene, pentylenecyclohexylene, pentylenecycloheptylene, pentylenecyclooctylene, pentylenecyclononylene, and pentylenecyclodecylene. -(C₁₋₁₀ alkylene)-(cyclo C₃₋₁₀ alkylene)- is, for example, methylene-(1,4-cyclohexylene). Examples of -(cyclo C₃₋₁₀ alkylene)-(C₁₋₁₀ alkylene)-include cyclopropylenemethylene, cyclobutylenemethylene, cyclopentylenemethylene, cyclohexylenemethylene, cycloheptylenemethylene, cyclooctylenemethylene, cyclononylenemethylene, cyclodecylenemethylene, cyclopropyleneethylene, cyclobutyleneethylene, cyclopentyleneethylene, cyclohexyleneethylene, cycloheptyleneethylene, cyclooctyleneethylene, cyclononyleneethylene, cyclodecyleneethylene, cyclopropylenepropylene, cyclobutylenepropylene, cyclopentylenepropylene, cyclohexylenepropylene, cycloheptylenepropylene, cyclooctylenepropylene, cyclononylenepropylene, cyclodecylenepropylene, cyclopropylenebutylene, cyclobutylenebutylene, cyclopentylenebutylene, cyclohexylenebutylene, cycloheptylenebutylene, cyclooctylenebutylene, cyclononylenebutylene, cyclodecylenebutylene, cyclopropylenepentylene, cyclobutylenepentylene, cyclopentylenepentylene, cyclohexylenepentylene, cycloheptylenepentylene, cyclooctylenepentylene, cyclononylenepentylene, and cyclodecylenepentylene.

As used herein, the term "C₂₋₄ alkylene" refers to a group having a -(CₘH₂ₘ)-structure. C₂₋₄ alkylene may be a straight chain or a branched chain, and is, for example, a straight chain. In this context, m is any integer of 2 to 4. Specific examples include ethylene, propylene, and butylene.

In one embodiment of the present invention, the compound of formula (I) is an 8- to 9-membered heterocyclic compound. As used herein, the term "8- to 9-membered heterocycle" refers to a heterocyclic group containing 8 to 9 atoms constituting a ring, including an N atom or an O atom as a hetero atom. In the present specification, "8- to 9-membered heterocycle" may have one triple bond.

**In** the present specification, the symbol • (black circle) in the chemical formulas indicates an attachment point.

As used herein, the terms "optionally substituted by" and "substituted by" refer to "optionally substituted by one substituent" and "substituted by one substituent," respectively, when the number of substituents (e.g., "one or more," "1 to 3," "1 or 2," "two," "one") is not clearly stated. For example, "B optionally substituted by A" and "B substituted by A" refer to "B optionally substituted by one A" and "B substituted by one A," respectively.

As used herein, the term "functional molecule" refers, without specific limitation, to a molecule that imparts a desired function when conjugated. Examples of the desired functions include, but are not limited to, a pharmacologically active function, a labeling function, a purification function, and a delivery function to a target site. Examples of "functional molecule" as used herein include a pharmacologically active compound, a label compound, a peptide, a protein, a nucleic acid, and a molecule used in a drug delivery system. Such a molecule may be a low molecular weight compound, a middle molecular weight compound, or a high molecular weight compound.

As used herein, the term "pharmacologically active compound" refers to a compound having a pharmacological activity. The pharmacologically active compound is, for example, a low molecular weight compound having a pharmacological activity.

As used herein, the term "label compound" refers to a compound labeled with a dye, a fluorescent substance, a tag, and/or a radioisotope. Among these, a compound labeled with a radioisotope represents a compound such as a low molecular weight compound, a middle molecular weight compound, or an antibody that was labeled with a radioactive isotope. The "label compound" may also be a dye, a fluorescent substance, a tag, or a radioactive isotope itself. The radioactive isotope may be coordinated to a chelating agent such as DOTA.

In the present specification, "peptide" may be any peptide exhibiting a useful function within the body.

In the present specification, "protein" may be any protein present in a living body or any protein exhibiting a useful function within the body. Examples include a protein having a pharmacological activity and a protein having an ability to recognize molecules. Specific examples may include an exportin protein or an importin protein, fibronectin, avidin, an antibody, and an enzyme.

In the present specification, "nucleic acid" may be any polymer of nucleotides. Specific examples include DNA and RNA. In the present specification, the nucleic acid may be modified. Examples of modifications include 2'-methoxyethyl (MOE) modification, incorporation of 2'-deoxynucleoside in RNA, internucleoside linkage via phosphorothioate, internucleoside linkage via phosphodiester, and conversion of cytosine to 5-methylcytosine.

In the present specification, a "linker attached to a functional molecule" may be a known linker. Examples include an amino acid linker (peptide linker), a fatty acid linker, a nucleic acid linker, a sugar-chain linker, and other chemical linkers (e.g., alkyl linker, PEG linker). The linker may also be, for example, a complex of a chemical linker and another linker such as a peptide linker.

As used herein, the term "drug delivery system" refers to a system for delivering an active ingredient into a target cell. Examples include a carrier such as a water-soluble macromolecule, a nano-sized microparticle (nanosphere), a liposome, and a micelle. A drug delivery system (DDS) molecule may further include a drug such as a low molecular weight compound, protein, peptide, nucleic acid, or a vaccine within the molecule.

As used herein, the term "conjugate" refers to a complex of functional molecules, the complex including two or more functional molecules. In this context, the functional molecules included in the "conjugate" may be identical functional molecules, or the "conjugate" may include a combination of different types of functional molecules.

In the present specification, R¹ of a compound represented by formula (I) may be a general protecting group such as an Fmoc group or a Boc group.

In the present specification, X⁴ of the compound represented by formula (I) may be a leaving group such as a p-nitrophenoxy group.

In the present specification, examples of salts of the compound represented by formula (I) include an acid addition salt and a base addition salt. Examples of the acid addition salt include a hydrochloride salt, a hydrobromide salt, a hydroiodide salt, a phosphate salt, a phosphonate salt, a sulfate salt, and the like; a sulfonate salt such as a methanesulfonate salt, an ethanesulfonate salt, a benzenesulfonate salt, and a p-toluenesulfonate salt; a carboxylate salt such as an acetate salt, a citrate salt, a malate salt, a tartrate salt, a succinate salt, a salicylate salt, a maleate salt, a fumarate salt, a benzoate salt, a malonate salt, a glycolate salt, an oxalate salt, a glucuronate salt, an adipate salt, a glutarate salt, a ketoglutarate salt, and a hippurate salt. Examples of the base addition salt include an alkali metal salt such as a sodium salt and a potassium salt; an alkaline earth metal salt such as a magnesium salt and a calcium salt; an ammonium salt such as an ammonium salt, an alkylammonium salt, a dialkylammonium salt, a trialkylammonium salt, and a tetraalkylammonium salt; an amino acid salt such as a lysine salt, an arginine salt, a glycine salt, a valine salt, a threonine salt, a serine salt, a proline salt, and an alanine salt. These salts are produced by contacting the compound with an acid or base that can be used to manufacture a medicament.

In the present specification, the compound represented by formula (I) or a salt thereof may be an anhydride or may form a solvate such as a hydrate. In this context, the term "solvate" refers to a solid formed by the complexation of the compound molecule with a solvent molecule. For example, when the solvent is water, the solvate is referred to as a hydrate. Examples of solvates other than hydrates include a solid that contains an alcohol (e.g., methanol, ethanol, n-propanol), N,N-dimethylformamide, or the like.

Furthermore, the compound represented by formula (I) and a salt thereof can exist in several tautomeric forms, such as a keto form and an enol form, an imine form and an enamine form, as well as mixtures thereof. The tautomers exist as a mixture of tautomers in solution. Generally, one type of tautomer is predominant in the sold form. Although one type of tautomer may be described, the present invention encompasses all tautomeric forms of the compound of the present invention.

The present invention includes all stereoisomers of the compound represented by formula (I) (e.g., enantiomer, diastereomer (including cis- and trans-geometric isomers)), a racemate of these isomers, and other mixtures. For example, the compound of the present invention may have one or more asymmetric points. The compounds of the present invention include a racemic mixture, a diastereomeric mixture, and an enantiomer of such compounds.

When the compound represented by formula (I) is obtained as a free form, the compound can be converted according to a routine procedure to a salt or a hydrate or solvate thereof that the compound may form.

When the compound represented by formula (I) is obtained as a salt, a hydrate, or a solvate of the compound, the compound can be converted according to a routine procedure to a free form thereof.

An element constituting the compound represented by formula (I) may be any isotope, and the present invention encompasses a compound of formula (I) containing an isotopic element. An isotopic compound is one in which at least one atom is replaced by an atom having the same atomic number (proton number) but a different mass number (total number of protons and neutrons). Examples of isotopes contained in the compound of the present invention include ²H, ³H, ¹³C, ¹⁴C, ¹⁵N, ¹⁷O, ¹⁸O, ³⁵S, ¹⁸F, and ³⁶Cl, which are isotopes of a hydrogen atom, a carbon atom, a nitrogen atom, an oxygen atom, a sulfur atom, a fluorine atom, and a chlorine atom, respectively. In particular, a radioisotope that emits radiation while decaying, such as ³H or ¹⁴C, is useful, for example, in a test to investigate the distribution of a medicament or compound within body tissue. A stable isotope does not decay, change in abundance, or emit radiation and can therefore be safely used. The isotopic compound of the present invention can be converted according to a routine procedure by replacing a reagent used for synthesis with a reagent containing the corresponding isotope.

In the present specification, when L of formula (V) is a halogen atom, the halogen atom is, for example, a chlorine atom. Examples of R¹⁰ of the compound represented by formula (V) include a hydrogen atom, methyl, acetyl, t-butyldimethylsilyl, trimethylsilyl, benzenesulfonyl, and methanesulfonyl. When X¹H of the compound represented by formula (V) is a hydroxy group, the hydroxy group may be protected by, for example, a silyl group such as TMS or TBS.

In one aspect, the compound represented by formula (I) can be used to form a 1,2,3-triazole ring through a 1,3-dipolar cycloaddition reaction with an azide compound (Example 6). Thus, the present invention provides a method for producing a conjugate of functional molecules by using a compound represented by formula (I) to form a 1,2,3-triazole ring.

In one embodiment, the reaction can be performed in a solvent, and examples of usable solvents include dimethyl sulfoxide, acetonitrile, and N,N-dimethylformamide. The temperature for performing the reaction is, for example, -78 to 50°C, and specifically 0 to 25°C. In one embodiment, the compound represented by formula (I) can be used, for example, in an amount of 0.1 to 10 equivalents, and specifically 0.5 to 1.5 equivalents, with respect to reactive azido groups. The reaction time of the reaction can be set, as appropriate, by those skilled in the art, and ranges, for example, from 10 minutes to 96 hours, and more specifically from 30 minutes to 2 hours.

In one aspect, a first functional molecule having a group represented by formula (II) can be used to form a conjugate through a 1,3-dipolar cycloaddition reaction with a second functional molecule containing an azido group represented by formula (III), the conjugate including functional molecules linked by a linker, the linker being represented by formula (IVa) or (IVb) and including a 1,2,3-triazole ring. In one embodiment, the reaction can be performed in a solvent. Examples of usable solvents include dimethyl sulfoxide, N,N-dimethylformamide, N-methylpyrrolidone, acetonitrile, methanol, ethanol, tetrahydrofuran, dioxane, dichloromethane, a mixed solvent of such a solvent and water. For example, dimethyl sulfoxide or N,N-dimethylformamide is used. The temperature for performing the reaction is, for example, 0 to 100°C, and specifically 0 to 25°C. In one embodiment, the first functional molecule can be used, for example, in an amount of 0.5 to 1.5 equivalents, and specifically 0.95 to 1.1 equivalents, with respect to reactive second functional molecules.

In one aspect, the compound represented by formula (I) can be obtained by cyclizing a compound represented by formula (V) by Nicholas reaction (Non Patent Literature 12) and then treating the resulting compound with ammonium hexanitratocerate (IV) or tetrabutylammonium fluoride. Thus, the present invention provides a method for producing a compound represented by formula (I), the method including the steps of cyclizing a compound represented by formula (V) by Nicholas reaction, and then treating the resulting compound with ammonium hexanitratocerate (IV) or tetrabutylammonium fluoride. In one embodiment, the Nicholas reaction can be performed in a solvent. Examples of usable solvents include dichloromethane, toluene, and tetrahydrofuran. For example, dichloromethane is used. In one embodiment, examples of a Lewis acid that can be used for the Nicholas reaction include a boron trifluoride-diethyl ether complex, diethylaluminum chloride, ethylaluminum dichloride, trimethylsilyl triflate, zinc chloride, and silver triflate. Examples of a Bronsted acid that can be used for the Nicholas reaction include p-toluenesulfonic acid, trifluoroacetic acid, trifluoromethanesulfonic acid, and methanesulfonic acid. The temperature for performing the Nicholas reaction is, for example, -78 to 50°C, and specifically 0 to 25°C. The reaction time of the Nicholas reaction can be set, as appropriate, by those skilled in the art, and ranges, for example, from 10 minutes to 24 hours, and more specifically from 30 minutes to 2 hours.

In one aspect, among the compounds represented by formula (I), an asymmetric compound can be provided as a single regioisomer of a 1,2,3-triazole ring through a 1,3-dipolar cycloaddition reaction with a sterically bulky azide compound (Example 7). In the conjugation process for PDC, among the compounds represented by formula (I), an asymmetric compound can be provided as a single regioisomer of a 1,2,3-triazole ring through a 1,3-dipolar cycloaddition reaction with a second functional molecule having a group represented by formula (III), such as a peptide or a nucleic acid. Accordingly, the compound of the present invention has the characteristic of being easily purified during production.

In one aspect, among the compounds represented by formula (I), a symmetric compound can form a 1,2,3-triazole ring without producing a plurality of regioisomers during the 1,3-dipolar cycloaddition reaction between an azide and an alkyne. Accordingly, the compound of the present invention has the characteristic of being easily purified during production.

In one aspect, an azide compound or the second functional molecule having a group represented by formula (III) is not limited to a specific type. A synthesized one may be used, or a commercially available one as a reagent for the 1,3-dipolar cycloaddition reaction between an azide and an alkyne may be used. Furthermore, the above described functional molecule into which an azido group was introduced may be used.

### EXAMPLES

Hereinafter, the present invention will be described in more detail with reference to synthesis examples as examples, but the present invention is not limited to these examples.

In the present specification, when, for example, a compound or a reagent is indicated by an abbreviation, each expression is based on abbreviations used by the IUPAC-IUB Commission on Biochemical Nomenclature or abbreviations commonly used in the art.

The meaning of abbreviations in the synthesis examples are as follows.
tert: tertiary
tBu: tertiary-butyl
Ac: acetyl
Boc: tertiary-butoxycarbonyl
Fmoc: 9-fluorenylmethyloxycarbonyl
Cbz: benzyloxycarbonyl
TMS: trimethylsilyl
TBS: tertiary-butyldimethylsilyl;
TIPS: triisopropylsilyl;
tBuSO₂: tertiary-butylsulphonyl
nBuLi: normal butyllithium
DMF: N,N-dimethylformamide
DMSO: dimethyl sulfoxide
NMP: N-methylpyrrolidone
DIPEA: diisopropylethylamine
EtOAc or AcOEt: ethyl acetate
Na₂SO₄: sodium sulfate
DCM: dichloromethane
THF: tetrahydrofuran
TBAF: tetrabutylammonium fluoride
BF₃·OEt₂: boron trifluoride-diethyl ether complex
Et₂AlCl: diethylaluminum chloride
EtAlCl₂: ethylaluminum dichloride
Ms: mesyl
Ns: nosyl
mCPBA: meta-chloroperbenzoic acid
MeOH: methanol
CAN: Cerium(IV) ammonium nitrate KOH: potassium hydroxide
LHMDS: lithium bis(trimethylsilyl)amide
CO: carbon monoxide
H₂O: water
TFA: trifluoroacetic acid
TsOH: p-toluenesulfonic acid
MsOH: methanesulfonic acid
Co₂(CO)₈: dicobalt octacarbonyl
HCl: hydrochloric acid
mL: milliliter (unit)
M: molar (mol/L) (unit)
mM: millimolar (unit)
mm: millimeter (unit)
nm: nanometer (unit)
µm: micrometer (unit)
Å: angstrom (unit)
min: minute (unit)
MS: mass spectrometry
mmol: millimole (unit)
g: gram (unit)
mg: milligram (unit)
NH silica gel: aminopropyl silica gel

Proton nuclear magnetic resonance (¹H-NMR) in the synthesis examples described below was measured in a deuterated chloroform solvent or a deuterated dimethyl sulfoxide solvent using JNM-ECP 300 manufactured by JEOL Ltd. or JNM-ECX 300 manufactured by JEOL Ltd., or Ascend TM500 manufactured by Bruker, unless specifically stated otherwise. The chemical shift was expressed as δ (ppm) relative to tetramethylsilane as the internal standard (0.0 ppm).

In the description of the NMR spectrum, "s" denotes a singlet, "d" denotes a doublet, "t" denotes a triplet, "q" denotes a quartet, "dd" denotes a doublet of doublets, "dt" denotes a doublet of triplets, "m" denotes a multiplet, "br" denotes a broad, "J" denotes a coupling constant, "Hz" denotes a hertz, "CDCl₃" denotes deuterated chloroform, and "DMSO-d₆" denotes deuterated dimethyl sulfoxide.

High performance liquid chromatography/mass spectrometry was performed using any of ACQUITY UPLC H-Class/QDa manufactured by Waters Corporation, ACQUITY UPLC H-Class/SQD2 manufactured by Waters Corporation, or LC-20AD/Triple Tof5600 manufactured by Shimadzu Corporation, unless specifically stated otherwise.

In the description of the high performance liquid chromatography/mass spectrometry, ESI+ denotes a positive mode of electrospray ionization, and (M + H)⁺ denotes a protonated ion. (M - MeOH + H)⁺ denotes an ion resulting from protonation and methanol elimination. (M - C₄H₈SO₂ + H)⁺ denotes an ion resulting from protonation and elimination of a tertiary-butylsulfonyl group. (M - C₅H₈O₂ + H)⁺ denotes an ion resulting from protonation and elimination of a tertiary-butoxycarbonyl group. (M - C₄H₈ + H)⁺ denotes an ion resulting from protonation and elimination of a tertiary-butyl group. (M - H₂O + H)⁺ denotes an ion resulting from protonation and water elimination.

In the description of the high performance liquid chromatography/mass spectrometry, ESI- denotes a negative mode of electrospray ionization, and M - H denotes an ion resulting from deprotonation.

### Analysis condition A

Column: Kinetex (R) 1.7 µm EVO C18 100 Å, 2.6 µm, 2.1 × 50 mm
Mobile phase A: 0.025% TFA in H₂O
Mobile phase B: 0.025% TFA in CH₃CN
Column temperature: 60°C
Gradient (% of B): 5% to 95% over 2.10 minutes, then held at 95% from 2.10 minutes to 2.84 minutes; Flow rate: 0.6 mL/min
Detection: UV 254 nm.

### [Example 1-1] Synthesis of tert-butyl 3,3-bis(hydroxymethyl)azetidine-1-carboxylate

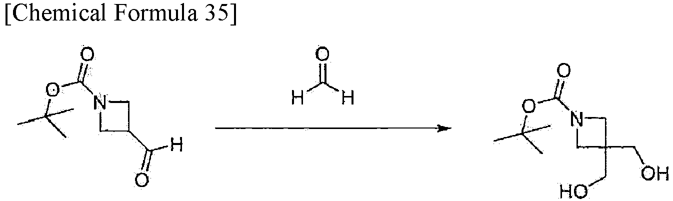

tert-Butyl 3-formylazetidine-1-carboxylate (3.88 g, 20.95 mmol, CAS: 177947-96-5) was dissolved in MeOH (105 mL). To this solution, a solution of KOH (4.88 g, 87 mmol) in H₂O (50 mL) and a 37% aqueous formaldehyde solution (17.2 mL, 230 mmol) were added. The resulting reaction mixture was stirred at room temperature for 150 minutes, and then stirred at 40°C for 24 hours. After MeOH was distilled off from the reaction mixture, a saturated aqueous ammonium chloride solution was added and extraction with EtOAc was performed. The obtained organic layer was dried over sodium sulfate, then filtered, and concentrated under reduced pressure. The obtained residue was purified by flash column chromatography using Isolera (Biotage; Sfaer HC Duo; mobile phase: a gradient from 3% to 15% MeOH in DCM). Subsequently, the fraction containing the target substance was concentrated under reduced pressure to obtain the title compound (3.71 g, 17.08 mmol) as white powder.

¹H-NMR (500 MHz, CDCl₃) δ 3.89 (s, 4H), 3.69 (s, 4H), 1.44 (s, 9H).

### [Example 1-2] Synthesis of tert-butyl 3,3-bis({N-[(tert-butoxy)carbonyl]methanesulfonamido}methyl)azetidine-1-carboxylate

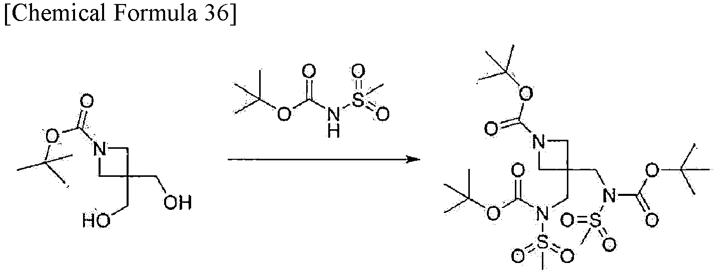

The tert-butyl 3,3-bis(hydroxymethyl)azetidine-1-carboxylate obtained in Example 1-1 (400 mg, 1.84 mmol) was dissolved in DCM (9.2 mL). To this solution, tert-butyl N-methanesulfonyl carbamate (755 mg, 3.87 mmol, CAS: 147751-16-4), triphenylphosphine (1062 mg, 4.05 mmol), and diisopropyl azodicarboxylate (2.42 mL, 4.60 mmol, 1.9 M solution in toluene, Tokyo Chemical Industry Co., Ltd.) were added under ice-cooling. The resulting reaction mixture was stirred at room temperature overnight. The reaction mixture was purified by flash column chromatography using Isolera (Biotage; Sfaer HC Duo; mobile phase: a gradient from 0% to 100% EtOAc in normal hexane). Subsequently, the fraction containing the target substance was concentrated under reduced pressure to obtain the title compound (740 mg, 1.29 mmol) as a colorless viscous substance.

Analysis condition A: Retention time: 2.00 min; ESI-MS (+); found m/z = 572.4 (M + H)⁺, theoretical m/z = 571.2.

### [Example 1-3] Synthesis of benzyl 3,3-bis(methanesulfonamidomethyl)azetidine-1-carboxylate

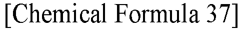

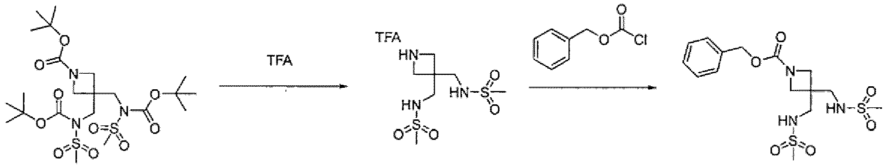

tert-Butyl 3,3-bis({N-[(tert-butoxy)carbonyl]methanesulfonamido}methyl) azetidine-1-carboxylate obtained in Example 1-2 (740 mg, 1.29 mmol) was dissolved in DCM (6.4 mL) and TFA (6.4 mL) was added. The resulting reaction mixture was stirred at room temperature for 2 hours. Toluene was added to the reaction mixture, and the mixture was concentrated and dried under reduced pressure. 400 mg from the 750 mg residue was dissolved in 1,4-dioxane/water (3.08 mL/0.34 mL), sodium carbonate (218 mg, 2.06 mmol) and benzyl chloroformate (140 mg, 0.822 mmol) were added under ice-cooling, and then stirred at room temperature for 3 hours. A saturated aqueous sodium bicarbonate solution was added to the reaction mixture, and extraction with DCM/MeOH was performed. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 0% to 100% EtOAc in normal hexane, followed by a gradient of 20% MeOH in DCM). Subsequently, the fraction containing the target substance was concentrated under reduced pressure to obtain the title compound (346 mg, 0.700 mmol) as a colorless viscous substance.

Analysis condition A: Retention time: 1.33 min; ESI-MS (+); found m/z = 406.3 (M + H)⁺, theoretical m/z = 405.1.

### [Example 1-4] Synthesis of benzyl 3-(methanesulfonamidomethyl)-{[N-(4-methoxybut-2-yn-1-yl)methanesulfonamido]methyl}azetidine-1-carboxylate

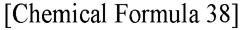

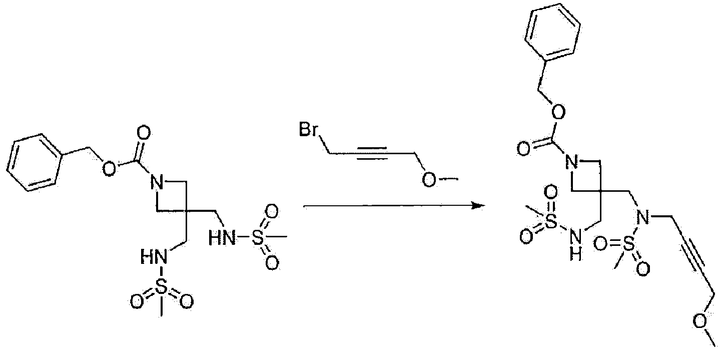

The benzyl 3,3-bis(methanesulfonamidomethyl)azetidine-1-carboxylate obtained in Example 1-3 (190 mg, 0.469 mmol) was dissolved in a mixed solvent of THF (1.9 mL) and DMF (0.47 mL). Sodium hydride (18.7 mg, 0.469 mmol, 60%, dispersed in liquid paraffin) was added under ice-cooling and stirred for 10 minutes without further processing. 1-Bromo-4-methoxy-2-butyne (76 mg, 0.469 mmol, CAS: 693-26-5, synthesized by the method described in the literature, The Journal of Organic Chemistry 2005, 70, 4059-4063) was added to the reaction mixture and stirred for 1 hour under ice-cooling. Water was added to the reaction mixture and extraction with EtOAc was performed. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 0% to 70% EtOAc in normal hexane). Subsequently, the fraction containing the target substance was concentrated under reduced pressure to obtain the title compound (70 mg, 0.144 mmol) as a colorless viscous substance.

Analysis condition A: Retention time: 1.62 min; ESI-MS (+); found m/z = 488.3 (M + H)⁺, theoretical m/z = 487.1.

### [Example 1-5] Synthesis of benzyl 3-(methanesulfonamidomethyl)-3-[(N-{[4-(methoxymethyl)-1,2-dicobaltatricyclo[1.1.0.0^{2,4}]butan-3-yl]methyl}methanesulfonamido)methyl]azetidine-1-carboxylate; hexakis(methanidylidyneoxidanium)

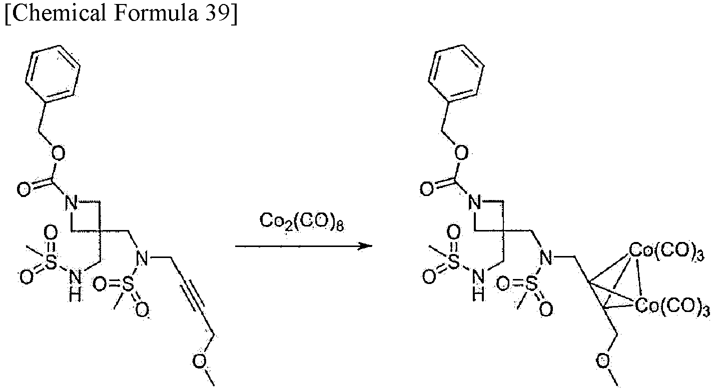

The benzyl 3-(methanesulfonamidomethyl)-{[N-(4-methoxybut-2-yn-1-yl)methanesulfonamido]methyl}azetidine-1-carboxylate obtained in Example 1-4 (66 mg, 0.135 mmol) was dissolved in DCM (0.68 mL) and Co₂(CO)₈ (51 mg, 0.149 mmol) was added. The resulting reaction mixture was stirred at room temperature for 2 hours. The reaction mixture was purified by flash column chromatography using Isolera (Biotage; Sfaer HC Duo; mobile phase: a gradient from 0% to 70% EtOAc in normal hexane). Subsequently, the fraction containing the target substance was concentrated under reduced pressure to obtain the title compound (86 mg, 0.111 mmol) as brown powder.

Analysis condition A: Retention time: 2.10 min; ESI-MS (+); found m/z = 742.2 (M - MeOH + H)⁺, theoretical m/z = 773.0.

### [Example 1-6] Synthesis of benzyl 3',7'-dimethanesulfonyl-3',7'-diaza-10',11'-dicobaltaspiro[azetidine-3,5'-tetracyclo[7.2.0.0^{1,10}.0^{9,11}]undecane]-1-carboxylate; hexakis(methanidylidyneoxidanium)

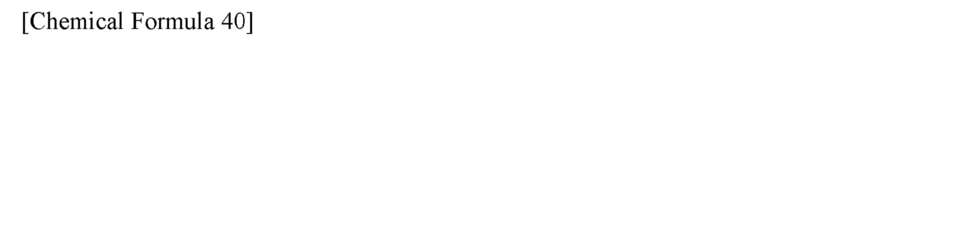

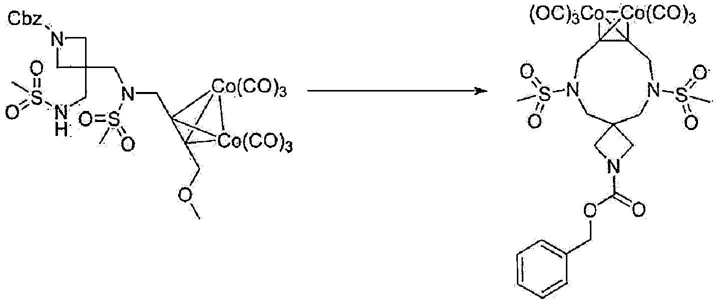

The benzyl 3-(methanesulfonamidomethyl)-3-[(N-{[4-(methoxymethyl)-1,2-dicobaltatricyclo[1.1.0.0^{2,4}]butan-3-yl]methyl}methanesulfonamido)methyl]azetidine-1-carboxylate; hexakis(methanidylidyneoxidanium) obtained in Example 1-5 (76 mg, 0.098 mmol) was dissolved in DCM (9.8 mL). A boron trifluoride-diethyl ether complex (0.037 mL, 0.295 mmol) was added and the resulting reaction mixture was stirred at room temperature for 1 hour. A saturated aqueous sodium bicarbonate solution was added to the reaction mixture, and extraction with EtOAc was performed. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 80% EtOAc in normal hexane). Subsequently, the fraction containing the target substance was concentrated under reduced pressure to obtain the title compound (52 mg, 0.070 mmol) as brown powder.

Analysis condition A: Retention time: 2.04 min; ESI-MS (+); found m/z = 742.2 (M + H)⁺, theoretical m/z = 741.0.

### [Example 1-7] Synthesis of benzyl 6,11-dimethanesulfonyl-2,6,11-triazaspiro[3.8]dodec-8-yn-2-carboxylate

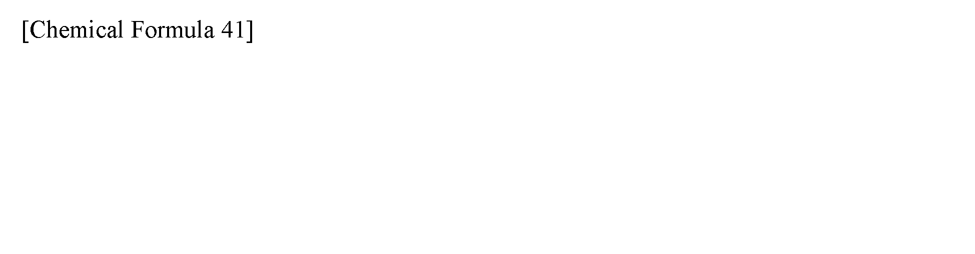

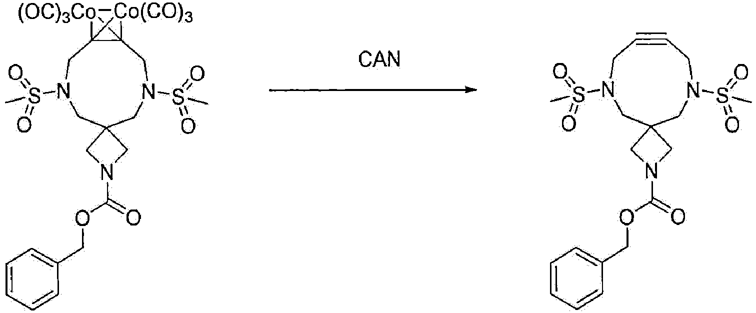

The benzyl 3',7'-dimethanesulfonyl-3',7'-diaza-10',11'-dicobaltaspiro[azetidine-3,5'-tetracyclo[7.2.0.0^{1,10}.0^{9,11}]undecane]-1-carboxylate; hexakis(methanidylidyneoxidanium) obtained in Example 1-6 (32 mg, 0.043 mmol) was suspended in diethyl ether (4.3 mL). Wakosil (R) C-200 (1 g, CAS: 63231-67-4) and CAN (237 mg, 0.432 mmol) were added under ice-cooling and the resulting reaction mixture was stirred at room temperature for 1 hour. The reaction mixture was passed through NH silica gel to filter off insoluble matter, and the target substance was eluted with EtOAc. The obtained organic layer was concentrated under reduced pressure. The obtained residue was purified by flash column chromatography using Isolera (Biotage; Sfaer HC Duo; mobile phase: 100% EtOAc). Subsequently, the fraction containing the target substance was concentrated under reduced pressure to obtain the title compound (10.1 mg, 0.022 mmol) as a colorless viscous substance.

Analysis condition A: Retention time: 1.53 min; ESI-MS (+); found m/z = 456.3 (M + H)⁺, theoretical m/z = 455.1.

¹H-NMR (500 MHz, CDCl₃) δ 7.38-7.29 (m, 5H), 5.10 (s, 2H), 4.03 (s, 4H), 3.98 (s, 4H), 3.63 (s, 4H), 2.89 (s, 6H).

### [Example 2-1] Synthesis of benzyl 3-hydroxy-3-(3-methoxyprop-1-yn-1-yl) azetidine-1-carboxylate

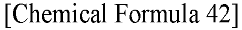

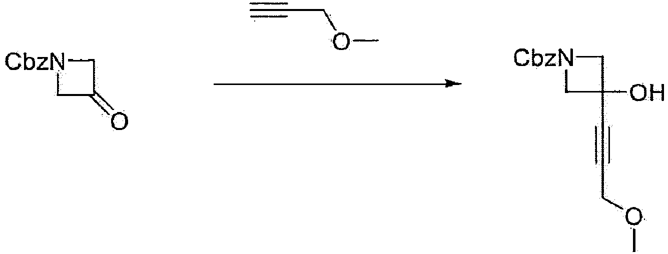

Methyl propargyl ether (2.05 g, 29.2 mmol, CAS: 627-41-8) was dissolved in THF (122 mL) and 2 M nBuLi (13.4 mL, 26.8 mmol, cyclohexane solution) was added at -78°C. The mixture was stirred for 20 minutes. Then, the mixture was allowed to warm to 0°C and stirred for 15 minutes. After cooling the mixture to -78°C again, a solution of benzyl 3-oxoazetidine-1-carboxylate (5.00 g, 24.36 mmol, CAS: 105258-93-3) in THF was added and stirred at -78°C for 1 hour. Then, the mixture was allowed to warm to 0°C and stirred for 30 minutes. Subsequently, the reaction was stopped with brine and the aqueous layer was extracted with EtOAc. The organic layer was washed with brine, then dried over sodium sulfate, filtered, and concentrated under reduced pressure. The obtained residue was purified by flash column chromatography using Isolera (Biotage; Sfaer HC Duo; mobile phase: a gradient from 10% to 70% EtOAc in normal hexane) to obtain the title compound (5.30 g, 19.3 mmol) as a colorless viscous substance.

Analysis condition A: Retention time: 1.54 min; ESI-MS (+); found m/z = 276.2 (M + H)⁺, theoretical m/z = 275.1.

### [Example 2-2] Synthesis of benzyl 3-(3-hydroxypropoxy)-3-(3-methoxyprop-1-yn-1-yl) azetidine-1-carboxylate

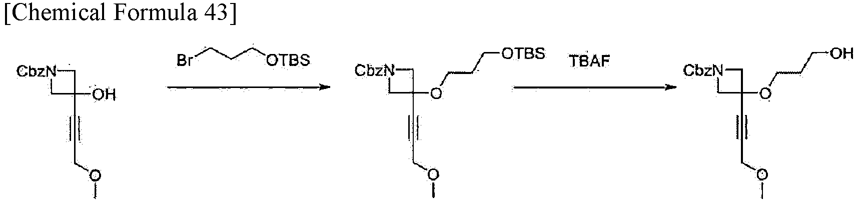

The benzyl 3-hydroxy-3-(3-methoxyprop-1-yn-1-yl)azetidine-1-carboxylate obtained in Example 2-1 (3.00 g, 10.9 mmol) was dissolved in THF (18 mL), and sodium hydride (0.654 g, 16.4 mmol, 60%, dispersed in fluid paraffin) was added at 0°C, and the mixture was stirred for 20 minutes. Then, a solution of (3-bromopropoxy) (tert-butyl) dimethylsilane (4.14 g, 16.4 mmol, CAS: 89031-84-5) in DMF (18 mL) was added at 0°C and stirred at room temperature overnight. Then, the reaction was quenched with a saturated aqueous ammonium chloride solution and the aqueous layer was subjected to the extraction with EtOAc. The organic layer was washed with brine, then dried over sodium sulfate, then filtered, and concentrated under reduced pressure. The resulting residue was dissolved in THF/water (9:1), and then TBAF·3H₂O (10.28 g, 32.7 mmol) was added and stirred overnight. The reaction mixture was dissolved in EtOAc and the aqueous layer was extracted withEtOAc. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 1% to 40% MeOH in DCM) to obtain the title compound (3.00 g, 9.00 mmol) as a light yellow viscous substance.

Analysis condition A: Retention time: 1.54 min; ESI-MS (+); found m/z = 334.4 (M + H)⁺, theoretical m/z = 333.2.

### [Example 2-3] Synthesis of benzyl 3-(3-hydroxypropoxy)-3-[4-(methoxymethyl)-1,2-dicobaltatricyclo[1.1.0.0^{2,4}]butan-3-yl]azetidine-1-carboxylate; hexakis(methanidylidyneoxidanium)

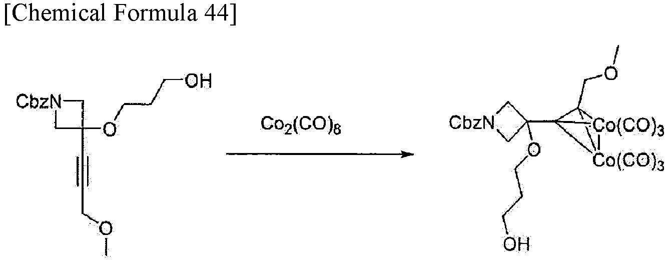

The benzyl 3-(3-hydroxypropoxy)-3-(3-methoxyprop-1-yn-1-yl)azetidine-1-carboxylate obtained in Example 2-2 (0.30 g, 0.90 mmol) was dissolved in DCM (9 mL), and Co₂(CO)₈ (0.40 g, 1.17 mmol) was added at room temperature. The mixture was stirred for 2 hours. After completion of the reaction, the mixture was concentrated under reduced pressure. The obtained residue was purified by flash column chromatography using Isolera (Biotage; Sfaer HC Duo; mobile phase: a gradient from 20% to 70% EtOAc in normal hexane) to obtain the title compound (0.49 g, 0.79 mmol) as a brown viscous substance.

Analysis condition A: Retention time: 2.16 min; ESI-MS (+); found m/z = 588.2 (M - MeOH + H)⁺, theoretical m/z = 619.0.

### [Example 2-4] Synthesis of benzyl 3',7'-dioxa-10',11'-dicobaltaspiro[azetidine-3,2'-tetracyclo[7.2.0.0^{1,10}.0^{9,11}]undecane]-1-carboxylate; hexakis(methanidylidyneoxidanium)

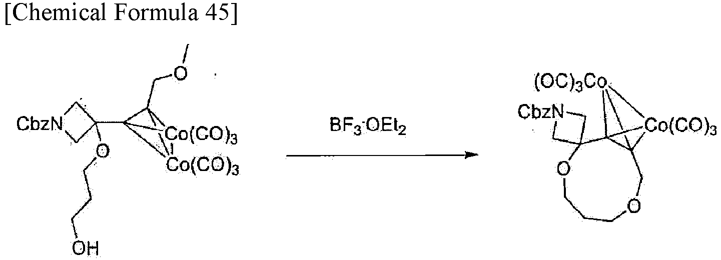

The benzyl 3-(3-hydroxypropoxy)-3-[4-(methoxymethyl)-1,2-dicobaltatricyclo[1.1.0.0^{2,4}]butan-3-yl]azetidine-1-carboxylate; hexakis(methanidylidyneoxidanium) obtained in Example 2-3 (0.46 g, 0.74 mmol) was dissolved in DCM (149 mL). A boron trifluoride-diethyl ether complex (0.14 mL, 1.11 mmol) was added at room temperature and the resulting reaction mixture was stirred for 30 minutes. The reaction was quenched by adding brine to the reaction mixture and the aqueous layer was subjected to the extraction with DCM. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 40% EtOAc in normal hexane) to obtain the title compound (0.174 g, 0.296 mmol) as a brown viscous substance.

Analysis condition A: Retention time: 2.25 min; ESI-MS (+); found m/z = 588.2 (M + H)⁺, theoretical m/z = 587.0.

### [Example 2-5] Synthetic of benzyl 5,9-dioxa-2-azaspiro[3.8]dodec-11-yn-2-carboxylate

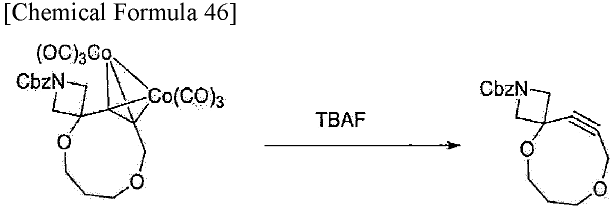

The benzyl 3',7'-dioxa-10',11'-dicobaltaspiro[azetidine-3,2'-tetracyclo[7.2.0.0^{1,10}.0^{9,11}]undecane]-1-carboxylate; hexakis(methanidylidyneoxidanium) obtained in Example 2-4 (0.027 g, 0.046 mmol) was dissolved in a mixed solvent (1.5 mL) of acetone and water (15:1), and TBAF·3H₂O (0.218 g, 0.690 mmol) was added at 0°C. After stirring at room temperature for 2 hours, water was added to the reaction mixture and the aqueous layer was extracted with EtOAc. The obtained organic layer was washed with brine, dried over sodium sulfate, then filtered, and concentrated under reduced pressure. The obtained residue was purified by flash column chromatography using Isolera (Biotage; Sfaer HC Duo; mobile phase: a gradient from 15% to 50% EtOAc in normal hexane) to obtain the title compound (0.011 g, 0.036 mmol) as a colorless viscous substance.

Analysis condition A: Retention time: 1.75 min; ESI-MS (+); found m/z = 302.2 (M + H)⁺, theoretical m/z = 301.1.

¹H-NMR (500 MHz, CDCl₃) δ 7.40-7.28 (m, 5H), 5.10 (s, 2H), 4.15 (s, 2H), 4.15-4.06 (m, 4H), 3.88-3.80 (m, 4H), 1.76-1.68 (m, 2H).

### [Example 3-1] Synthesis of benzyl 3-(3-methoxyprop-1-yn-1-yl)-3-[(2-methylpropane-2-sulfinyl)amino]azetidine-1-carboxylate

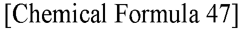

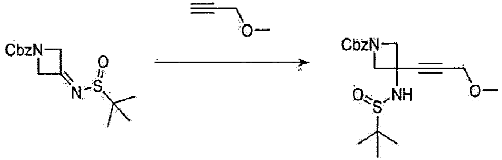

Methyl propargyl ether (0.59 g, 8.43 mmol, CAS: 627-41-8) was dissolved in THF (65 mL) and 2 M nBuLi (3.9 mL, 7.78 mmol, cyclohexane solution) was added at -78°C. The mixture was allowed to warm to 0°C and stirred for 10 minutes. To the reaction mixture, was added a solution of benzyl 3-[(2-methylpropane-2-sulfinyl)imino]azetidine-1-carboxylate (2.00 g, 6.49 mmol, CAS: 1638764-74-5, synthesized by a method described in US 2020/0038378 A1). The mixture was stirred at 0°C for 20 minutes, and the reaction was quenched with a saturated aqueous ammonium chloride solution. After the aqueous layer was extracted with EtOAc, the obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 50% to 100% EtOAc in normal hexane) to obtain the title compound (0.96 g, 2.54 mmol) as a yellow viscous substance.

Analysis condition A: Retention time: 1.72 min; ESI-MS (+); found m/z = 379.3 (M + H)⁺, theoretical m/z = 378.2.

### [Example 3-2] Synthesis of benzyl 3-({3-[(tert-butyldimethylsilyl)oxy]propyl}(2-methylpropane-2-sulfinyl)amino)-3-(3-methoxyprop-1-yn-1-yl)azetidine-1-carboxylate

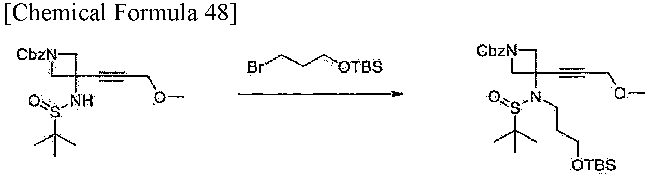

Sodium hydride (42.3 mg, 60%, dispersed in liquid paraffin) was suspended in THF (2.6 mL) at 0°C. Then, the benzyl 3-(3-methoxyprop-1-yn-1-yl)-3-[(2-methylpropane-2-sulfinyl)amino]azetidine-1-carboxylate obtained in Example 3-1 (0.200 g, 0.528 mmol) was added at 0°C and stirred for 10 minutes. A solution (2.6 mL) of (3-bromopropoxy) (tert-butyl) dimethylsilane (0.294 g, 1.16 mmol, CAS: 89031-84-5) in DMF was added to the reaction mixture and stirred at room temperature overnight. After the reaction mixture was cooled to 0°C, the reaction was quenched with a saturated aqueous ammonium chloride solution and the aqueous layer was extracted with EtOAc. Subsequently, the obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 100% EtOAc in normal hexane) to obtain the title compound (0.080 g, 0.145 mmol) as a colorless viscous substance.

Analysis condition A: Retention time: 2.37 min; ESI-MS (+); found m/z = 551.4 (M + H)⁺, theoretical m/z = 550.3.

### [Example 3-3] Synthesis of benzyl 3-(N-{3-[(tert-butyldimethylsilyl)oxy]propyl}-2-methylpropane-2-sulfonamido)-3-(3-methoxyprop-1-yn-1-yl)azetidine-1-carboxylate

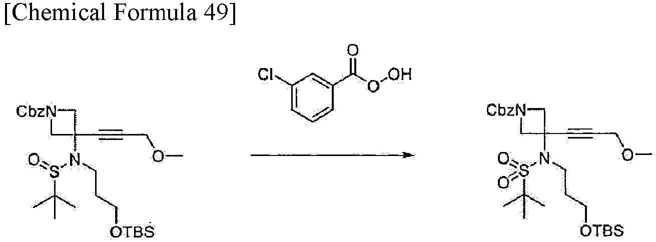

The benzyl 3-({3-[(tert-butyldimethylsilyl)oxy]propyl}(2-methylpropane-2-sulfinyl)amino)-3-(3-methoxyprop-1-yn-1-yl)azetidine-1-carboxylate obtained in Example 3-2 (0.080 g, 0.145 mmol) was dissolved in DCM (1.5 mL), and mCPBA containing 30% water (54 mg, 0.218 mmol, CAS: 937-14-4) was added at room temperature. The mixture was stirred for 2 hours. After the reaction mixture was cooled to 0°C, the reaction was quenched with a brine and the aqueous layer was extracted with EtOAc. Subsequently, the obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 50% EtOAc in normal hexane) to obtain the title compound (0.068 g, 0.12 mmol) as a colorless viscous substance.

Analysis condition A: Retention time: 2.45 min; ESI-MS (+); found m/z = 447.4 (M - C₄H₈SO₂ + H)⁺, theoretical m/z = 566.3.

### [Example 3-4] Synthesis of benzyl 3-[N-(3-hydroxypropyl)-2-methylpropane-2-sulfonamido]-3-(3-methoxyprop-1-yn-1-yl)azetidine-1-carboxylate

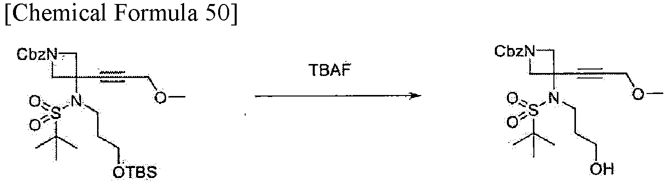

After the benzyl 3-(N-{3-[(tert-butyldimethylsilyl)oxy]propyl}-2-methylpropane-2-sulfonamido)-3-(3-methoxyprop-1-yn-1-yl)azetidine-1-carboxylate obtained in Example 3-3 (0.068 g, 0.12 mmol) was dissolved in THF (1.5 mL), 1 M TBAF (0.18 mL, 0.18 mmol, THF solution) was added at room temperature and stirred for 3 hours. The reaction mixture was concentrated under reduced pressure. The obtained residue was purified by flash column chromatography using Isolera (Biotage; Sfaer HC Duo; mobile phase: a gradient from 30% to 100% EtOAc in normal hexane) to obtain the title compound (0.053 g, 0.117 mmol) as a colorless viscous substance.

Analysis condition A: Retention time: 1.71 min; ESI-MS (+); found m/z = 333.3 (M - C₄H₈SO₂ + H)⁺, theoretical m/z = 452.2.

### [Example 3-5] Synthesis of benzyl 3'-(2-methylpropane-2-sulfonyl)-7'-oxa-3'-aza-10',11'-dicobaltaspiro[azetidine-3,2'-tetracyclo[7.2.0.0^{1,10}.0^{9,11}]undecane]-1-carboxylate; hexakis(methanidylidyneoxidanium)

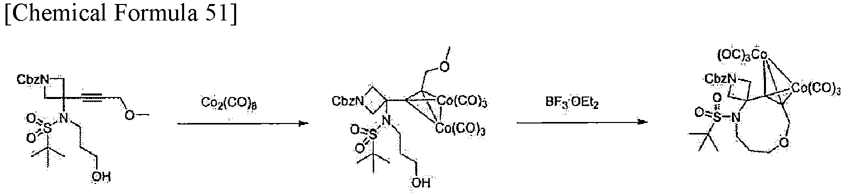

The benzyl 3-[N-(3-hydroxypropyl)-2-methylpropane-2-sulfonamido]-3-(3-methoxyprop-1-yn-1-yl)azetidine-1-carboxylate obtained in Example 3-4 (0.053 g, 0.117 mmol) was dissolved in DCM (1.1 mL), and Co₂(CO)₈ (0.060 g, 0.176 mmol) was added at room temperature. The mixture was stirred for 3 hours. After completion of the reaction, the mixture was concentrated under reduced pressure. The obtained residue was purified by flash column chromatography using Isolera (Biotage; Sfaer HC Duo; mobile phase: a gradient from 30% to 80% EtOAc in normal hexane) to obtain, as a brown viscous substance, benzyl 3-[N-(3-hydroxypropyl)-2-methylpropane-2-sulfonamido]-3-[4-(methoxymethyl)-1,2-dicobaltatricyclo[1.1.0.0^{2,4}]butan-3-yl]azetidine-1-carboxylate; hexakis(methanidylidyneoxidanium) (0.082 g, 0.111 mmol). The above obtained benzyl 3-[N-(3-hydroxypropyl)-2-methylpropane-2-sulfonamido]-3-[4-(methoxymethyl)-1,2-dicobaltatricyclo[1.1.0.0^{2,4}]butan-3-yl]azetidine-1-carboxylate; hexakis(methanidylidyneoxidanium) (0.020 g, 0.027 mmol) was dissolved in DCM (5 mL). A boron trifluoride-diethyl ether complex (0.005 mL, 0.041 mmol) was added at room temperature and the resulting reaction mixture was stirred for 15 minutes. The reaction was quenched with a brine to the reaction mixture and the aqueous layer was extracted with DCM. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 50% EtOAc in normal hexane) to obtain the title compound (0.013 g, 0.027 mmol) as a brown viscous substance.

Analysis condition A: Retention time: 2.29 min; ESI-MS (+); found m/z = 707.1 (M + H)⁺, theoretical m/z = 706.0.

### [Example 3-6] Synthesis of benzyl 5-(2-methylpropane-2-sulfonyl)-9-oxa-2,5-diazaspiro[3.8]dodec-11-yn-2-carboxylate

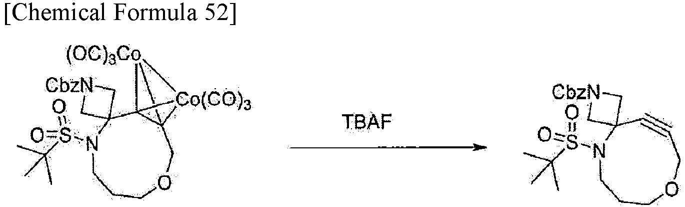

The benzyl 3'-(2-methylpropane-2-sulfonyl)-7'-oxa-3'-aza-10',11'-dicobaltaspiro[azetidine-3,2'-tetracyclo[7.2.0.0^{1,10}.0^{9,11}]undecane]-1-carboxylate; hexakis(methanidylidyneoxidanium) obtained in Example 3-5 (0.013 g, 0.018 mmol) was dissolved in a mixed solvent (0.36 mL) of acetone and water (15:1), and then TBAF·3H₂O (0.115 g, 0.365 mmol) was added at room temperature. After stirring at room temperature for 3 hours, water was added to the reaction mixture and the aqueous layer was extracted with EtOAc. The obtained organic layer was washed with brine, dried over sodium sulfate, then filtered, and concentrated under reduced pressure. The obtained residue was purified by flash column chromatography using Isolera (Biotage; Sfaer HC Duo; mobile phase: a gradient from 20% to 70% EtOAc in normal hexane) to obtain the title compound (0.005 g, 0.012 mmol) as white powder.

Analysis condition A: Retention time: 1.84 min; ESI-MS (+); found m/z = 421.3 (M + H)⁺, theoretical m/z = 420.2.

¹H-NMR (500 MHz, CDCl₃) δ 7.39-7.30 (m, 5H), 5.11 (s, 2H), 4.52-4.46 (m, 2H), 4.27-4.19 (m, 4H), 3.90-3.84 (m, 2H), 3.53-3.45 (m, 2H), 2.11-2.03 (m, 2H), 1.41 (s, 9H).

### [Example 4-1] Synthesis of benzyl 3-(3-methanesulfonamidopropoxy)-3-[4-(methoxymethyl)-1,2-dicobaltatricyclo[1.1.0.0^{2,4}]butan-3-yl]azetidine-1-carboxylate; hexakis(methanidylidyneoxidanium)

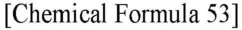

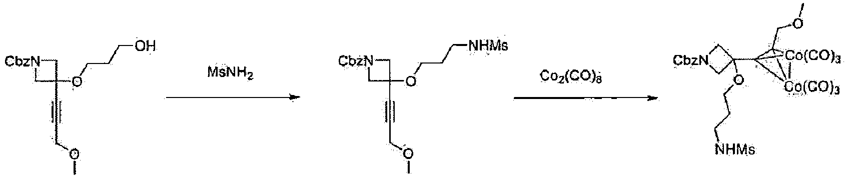

The benzyl 3-(3-hydroxypropoxy)-3-(3-methoxyprop-1-yn-1-yl)azetidine-1-carboxylate obtained in Example 2-2 (0.12 g, 0.360 mmol) was dissolved in toluene (1.5 mL), and MsNH₂ (0.103 g, 1.08 mmol) and cyanomethylenetributylphosphorane (0.261 g, 1.08 mmol, CAS: 157141-27-0) were added. The mixture was stirred at room temperature overnight. After completion of the reaction, the mixture was concentrated under reduced pressure. The obtained residue was purified by flash column chromatography using Isolera (Biotage; Sfaer HC Duo; mobile phase: a gradient from 40% to 80% EtOAc in normal hexane) to obtain an intermediate as a red viscous substance. The intermediate was dissolved in DCM (1.8 mL), and Co₂(CO)₈ (0.148 g, 0.433 mmol) was added at room temperature. The mixture was stirred for 30 minutes. After completion of the reaction, the mixture was concentrated under reduced pressure. The obtained residue was purified by flash column chromatography using Isolera (Biotage; Sfaer HC Duo; mobile phase: a gradient from 30% to 50% EtOAc in normal hexane) to obtain the title compound (0.196 g, 0.281 mmol) as a brown viscous substance.

Analysis condition A: Retention time: 2.27 min; ESI-MS (+); found m/z = 697.1 (M + H)⁺, theoretical m/z = 696.0.

### [Example 4-2] Synthesis of benzyl 7'-methanesulfonyl-3'-oxa-7'-aza-10',11'-dicobaltaspiro[azetidine-3,2'-tetracyclo[7.2.0.0^{1,10}.0^{9,11}]undecane]-1-carboxylate; hexakis(methanidylidyneoxidanium)

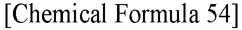

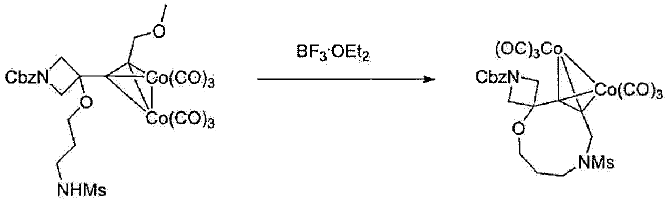

The benzyl 3-(3-methanesulfonamidopropoxy)-3-[4-(methoxymethyl)-1,2-dicobaltatricyclo[1.1.0.0^{2,4}]butan-3-yl]azetidine-1-carboxylate; hexakis(methanidylidyneoxidanium) obtained in Example 4-1 (0.100 g, 0.144 mmol) was dissolved in DCM (57 mL). A boron trifluoride-diethyl ether complex (0.152 mL, 1.21 mmol) was added at 0°C and the resulting reaction mixture was stirred for 1 hour. The reaction was quenched with a brine to the reaction mixture and the aqueous layer was extracted with DCM. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 60% EtOAc in normal hexane) to obtain the title compound (0.093 g, 0.140 mmol) as a reddish brown viscous substance.

Analysis condition A: Retention time: 2.19 min; ESI-MS (+); found m/z = 665.1 (M + H)⁺, theoretical m/z = 664.0.

### [Example 4-3] Synthesis of benzyl 9-methanesulfonyl-5-oxa-2,9-diazaspiro[3.8]dodec-11-yn-2-carboxylate

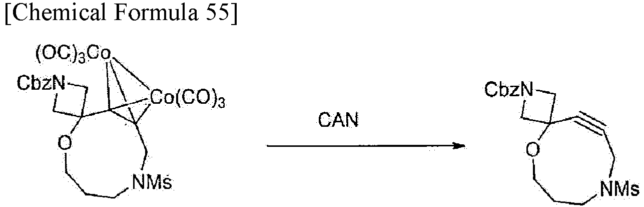

The benzyl 7'-methanesulfonyl-3'-oxa-7'-aza-10',11'-dicobaltaspiro[azetidine-3,2'-tetracyclo[7.2.0.0^{1,10}.0^{9,11}]undecane]-1-carboxylate; hexakis(methanidylidyneoxidanium) obtained in Example 4-2 (0.200 g, 0.301 mmol) was dissolved in diethyl ether (30 mL). Wakosil (R) C-200 (5 g) was added, followed by the addition of CAN (1.65 g, 3.01 mmol) at 0°C. After stirring at 0°C for 1 hour, the mixture was allowed to warm to room temperature and was stirred for 1 hour. After completion of the reaction, the reaction mixture was passed through NH silica gel to filter off insoluble matter. Subsequently, the filtrate was concentrated under reduced pressure. The obtained residue was purified by flash column chromatography using Isolera (Biotage; Sfaer HC Duo; mobile phase: a gradient from 30% to 80% EtOAc in normal hexane, followed by a gradient from 1% to 20% MeOH in DCM) to obtain the title compound (0.010 g, 0.036 mmol) as a light yellow solid.

Analysis condition A: Retention time: 1.71 min; ESI-MS (+); found m/z = 379.3 (M + H)⁺, theoretical m/z = 378.1.

¹H-NMR (500 MHz, CDCl₃) δ 7.40-7.29 (m, 5H), 5.10 (s, 2H), 4.14-4.03 (m, 4H), 3.96 (s, 2H), 3.91-3.84 (m, 2H), 3.46-3.38 (m, 2H), 2.84 (s, 3H), 1.95-1.84 (m, 2H).

### [Example 5-1] Synthesis of (2S)-6-(2-bromo-2-methylpropanamido)-2- {[(tert-butoxy)carbonyl]amino}hexanoic acid

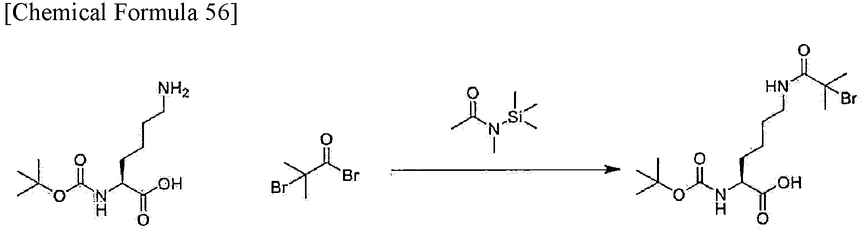

N-Boc-L-lysine (1.13 g, 4.57 mmol, CAS: 13734-28-6) was suspended in THF (11.6 mL) and N-methyl-N-trimethylsilylacetamide (1.53 mL, 9.57 mmol) was added. The resulting reaction mixture was stirred at 50°C for 1 hour. The reaction mixture was cooled to 0°C. DIPEA (0.89 mL, 5.22 mmol) and 2-bromoisobutyrylbromide (1.00 g, 4.35 mmol, CAS: 20769-85-1) were added and the mixture was stirred for 1 hour. The reaction was quenched with a 10% aqueous sodium carbonate solution, and the aqueous layer was washed twice with heptane. The aqueous phase was acidified by adding 1M aqueous hydrochloric acid solution and was extracted with EtOAc. The obtained organic layer was washed with brine, then dried over sodium sulfate, then filtered, and concentrated under reduced pressure to obtain the title compound (1.64 g, 4.15 mmol) as white powder.

Analysis condition A: Retention time: 1.62 min; ESI-MS (+); found m/z = 295.1 (M - C₅H₈O₂ + H)⁺, theoretical m/z = 394.1.

### [Example 5-2] Synthesis of (2S)-6-(2-azido-2-methylpropanamido)-2- {[(tert-butoxy)carbonyl]amino}hexanoic acid

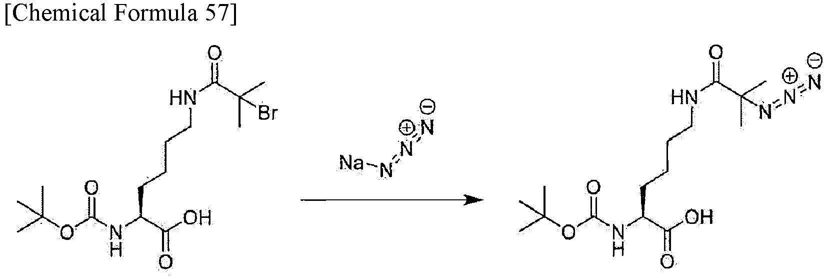

The (2S)-6-(2-bromo-2-methylpropanamido)-2-{[(tert-butoxy)carbonyl]amino}hexanoic acid obtained in Example 5-1 (200 mg, 0.506 mmol) was dissolved in DMSO (1.7 mL). Sodium azide (66 mg, 1.01 mmol) was added and the resulting reaction mixture was stirred at 50°C for 3 hours. Water was added to the reaction mixture and extraction with EtOAc was performed. The obtained organic layer was washed with a 1 N aqueous hydrochloric acid solution and brine, then dried over sodium sulfate, then filtered, and concentrated under reduced pressure to obtain the title compound (146 mg, 0.408 mmol) as a colorless viscous substance.

¹H-NMR (500 MHz, CDCl₃) δ 6.62-6.56 (m, 1H), 5.23 (d, J=7.5Hz, 1H), 4.30-4.22 (m, 1H), 3.32-3.17 (m, 2H), 1.95-1.85 (m, 1H), 1.77-1.69 (m, 1H), 1.58-1.55 (m, 1H), 1.53 (s, 6H), 1.45 (s, 9H), 1.44-1.32 (m, 3H)

### [Example 5-3] Synthesis of (2S)-6-(2-azido-2-methylpropanamido)-2-({[(9H-fluoren-9-yl)methoxy]carbonyl}amino)hexanoic acid

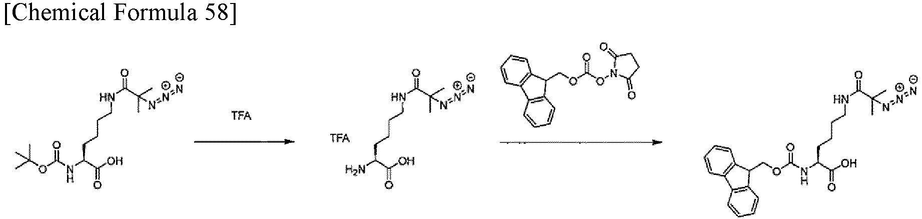

The (2S)-6-(2-azido-2-methylpropanamido)-2-{[(tert-butoxy)carbonyl]amino}hexanoic acid obtained in Example 5-2 (146 mg, 0.408 mmol) was cooled on ice. TFA (1.36 mL) was added and the resulting reaction mixture was stirred for 2 hours without further processing. Toluene was added to the reaction mixture, and the mixture was concentrated and dried under reduced pressure. The residue was dissolved in acetone/water (1.0 mL/1.0 mL), and sodium bicarbonate (274 mg, 3.26 mmol) and N-[(9H-fluoren-9-ylmethoxy)carbonyloxy]succinimide (151 mg, 0.448 mmol, CAS: 82911-69-1) were added. The resulting reaction mixture was stirred at room temperature overnight. EtOAc was added to the reaction mixture and extraction was performed. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: 100% EtOAc, followed by a gradient from 1% to 20% MeOH in DCM). Subsequently, the fraction containing the target substance was concentrated under reduced pressure to obtain the title compound (148 mg, 0.309 mmol) as white powder.

Analysis condition A: Retention time: 1.68 min; ESI-MS (+); found m/z = 480.3 (M + H)⁺, theoretical m/z = 479.2.

¹H-NMR (500 MHz, CDCl₃) δ 7.77-7.73 (m, 2H), 7.62-7.52 (m, 2H), 7.42-7.37 (m, 2H), 7.32-7.26 (m, 2H), 6.68-6.52 (m, 1H), 5.73-5.62 (m, 1H), 4.52-4.15 (m, 4H), 3.32-3.17 (m, 2H), 1.98-1.87 (m, 1H), 1.83-1.72 (m, 1H), 1.58-1.47 (m, 7H), 1.46-1.28 (m, 3H).

[Example 6] Synthesis of (2S)-6-{ 1'-[(benzyloxy)carbonyl]-5,9-dimethanesulfonyl-4,5,6,8,9,10-hexahydro-1H-spiro[[1,2,3]triazolo[4,5-g][1,5]diazonine-7,3'-azetidin]-1-yl}-2-({[(9H-fluoren-9-yl)methoxy]carbonyl}amino)hexanoic acid

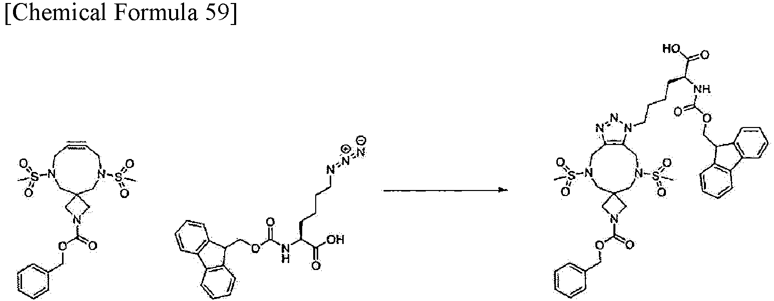

The benzyl 6,11-dimethanesulfonyl-2,6,11-triazaspiro[3.8]dodec-8-yn-2-carboxylate obtained in Example 1-7 (13.5 mg, 0.030 mmol) was dissolved in DMSO (0.59 mL). (2S)-6-azido-2-({[(9H-fluoren-9-yl)methoxy]carbonyl}amino)hexanoic acid (12.9 mg, 0.033 mmol, CAS: 159610-89-6) was added and the resulting reaction mixture was stirred at room temperature for 30 hours. Water was added to the reaction mixture and extraction with EtOAc was performed. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: 100% EtOAc, followed by a gradient from 0% to 30% MeOH in DCM). Subsequently, the fraction containing the target substance was concentrated under reduced pressure to obtain the title compound (21 mg, 0.025 mmol) as white powder.

Analysis condition A: Retention time: 1.90 min; ESI-MS (+); found m/z = 850.6 (M + H)⁺, theoretical m/z = 849.3.

¹H-NMR (500 MHz, DMSO-d₆) δ 12.83-12.34 (m, 1H), 7.91-7.87 (m, 2H), 7.73-7.67 (m, 2H), 7.44-7.39 (m, 2H), 7.38-7.28 (m, 7H), 5.01 (s, 2H), 4.63-4.53 (m, 4H), 4.36-4.19 (m, 5H), 3.79-3.58 (m, 7H), 3.26-3.24 (m, 2H), 3.18 (s, 3H), 3.04 (s, 3H), 1.82-1.66 (m, 3H), 1.66-1.56 (m, 1H), 1.35-1.21 (m, 2H).

This result indicates that benzyl 6,11-dimethanesulfonyl-2,6,11-triazaspiro[3.8]dodec-8-yn-2-carboxylate, which is a compound of the present invention, undergoes a click reaction with an azide compound in the same manner as an alkyne used in known click chemistry.

### [Example 7] Synthesis of (2S)-6-(2-{1'-[(benzyloxy)carbonyl]-6,7,8,10-tetrahydro-1H spiro[[1,5]dioxonino[7,8-d][1,2,3]triazol-4,3'-azetidin]-1-yl}-2-methylpropanamido)-2-({[(9H-fluoren-9-yl)methoxy]carbonyl}amino)hexanoic acid

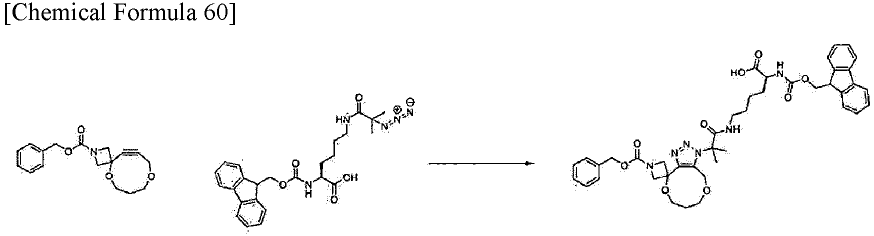

The benzyl 5,9-dioxa-2-azaspiro[3.8]dodec-11-yn-2-carboxylate obtained in Example 2-5 (13.0 mg, 0.043 mmol) was dissolved in DMSO (0.78 mL), and (2S)-6-(2-azido-2-methylpropanamido)-2-({[(9H-fluoren-9-yl)methoxy]carbonyl}amino)hexanoic acid obtained in Example 5-3 (18.8 mg, 0.033 mmol) was added. The resulting reaction mixture was stirred at room temperature for 18 hours. Water was added to the reaction mixture and extraction with EtOAc/Et₂O (3: 1) was performed. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 0% to 30% MeOH in DCM). Subsequently, the fraction containing the target substance was concentrated under reduced pressure to obtain the title compound (23.2 mg, 0.030 mmol) as white powder.

Analysis condition A: Retention time: 2.04 min; ESI-MS (+); found m/z = 781.6 (M + H)⁺, theoretical m/z = 780.4.

¹H-NMR (500 MHz, DMSO-d₆) δ 12.57-12.52 (m, 1H), 7.91-7.85 (m, 3H), 7.74-7.70 (m, 2H), 7.61-7.58 (m, 1H), 7.44-7.39 (m, 2H), 7.38-7.30 (m, 7H), 5.08 (s, 2H), 4.50 (s, 2H), 4.44-4.29 (m, 2H), 4.29-4.07 (m, 5H), 3.93-3.85 (m, 1H), 3.75-3.70 (m, 2H), 3.38-3.33 (m, 2H), 3.06-2.99 (m, 2H), 1.79 (s, 6H), 1.71-1.52 (m, 4H), 1.43-1.21 (m, 4H).

This result indicates that benzyl 5,9-dioxa-2-azaspiro[3.8]dodec-11-yn-2-carboxylate, which is a compound of the present invention, undergoes a click reaction with an azide compound in the same manner as an alkyne used in known click chemistry.

### [Example 8-1] Synthesis of tert-butyl 3-hydroxy-3-(3-methoxyprop-1-yn-1-yl)azetidine-1-carboxylate

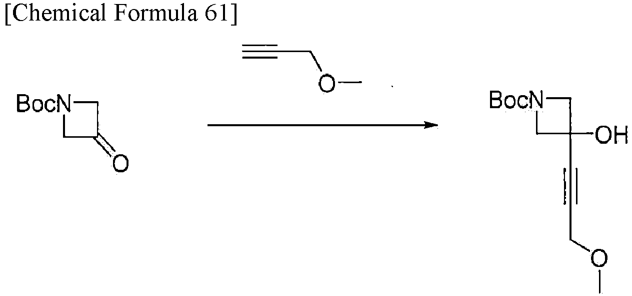

Methyl propargyl ether (4.26 g, 60.7 mmol, CAS: 627-41-8) was dissolved in THF (156 mL) and 2 M nBuLi (28.0 mL, 56.1 mmol, cyclohexane solution) was added at -78°C. The mixture was stirred for 10 minutes. Then, the mixture was allowed to warm to 0°C and stirred for 10 minutes. After cooling the mixture to -78°C again, a solution of tert-butyl 3-oxoazetidine-1-carboxylate (8.00 g, 46.7 mmol, CAS: 398489-26-4) in THF was added and stirred at -78°C for 20 minutes. Then, the mixture was allowed to warm to 0°C and stirred for 30 minutes. Subsequently, the reaction was quenched with a saturated ammonium chloride solution at -78°C and the aqueous layer was extracted with EtOAc. The organic layer was washed with brine, then dried over sodium sulfate, filtered, and concentrated under reduced pressure. The obtained residue was purified by flash column chromatography using Isolera (Biotage; Sfaer HC Duo; mobile phase: a gradient from 20% to 50% EtOAc in normal hexane) to obtain the title compound (11.28 g, 45.7 mmol) as a white yellow viscous substance.

Analysis condition A: Retention time: 1.35 min; ESI-MS (+); found m/z = 242.2 (M + H)⁺, theoretical m/z = 241.1.

¹H-NMR (500 MHz, CDCl₃) δ 4.21-4.17 (m, 2H), 4.15 (s, 2H), 4.05-4.01 (m, 2H), 3.39 (s, 3H), 2.77 (s, 1H), 1.44 (s, 9H).

### [Example 8-2] Synthesis of tert-butyl 3-(3-hydroxypropoxy)-3-(3-methoxyprop-1-yn-1-yl)azetidine-1-carboxylate

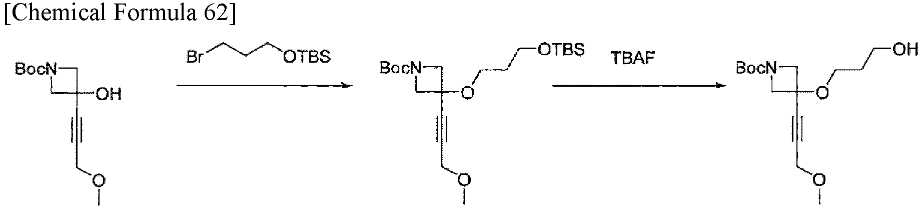

Sodium hydride (2.56 g, 64.0 mmol, 60%, dispersed in liquid paraffin) was added to DMF (76 mL), and a solution of the tert-butyl 3-hydroxy-3-(3-methoxyprop-1-yn-1-yl)azetidine-1-carboxylate obtained in Example 8-1 (11.03 g, 45.7 mmol) in THF (76 mL) was added at 0°C. The mixture was stirred for 20 minutes. Then, a solution of (3-bromopropoxy)(tert-butyl)dimethylsilane (17.37 g, 68.6 mmol, CAS: 89031-84-5) in DMF was added at 0°C and the mixture was stirred at room temperature for 6 hours. Then, the reaction was quenched with a saturated aqueous ammonium chloride solution and the aqueous layer was extracted with EtOAc. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 0% to 30% EtOAc in normal hexane) to obtain an intermediate as a colorless transparent viscous substance. The intermediate obtained above was added to a THF solution (147 mL) and 1 M TBAF (66.1 mL, 66.1 mmol) was added at 0°C. The mixture was stirred at room temperature for 3 hours. After completion of the reaction, the reaction was quenched with a saturated aqueous ammonium chloride solution, and then, the aqueous layer was extracted with EtOAc. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 0% to 80% EtOAc in normal heptane) to obtain the title compound (13.05 g, 43.6 mmol) as a colorless transparent viscous substance.

Analysis condition A: Retention time: 1.51 min; ESI-MS (+); found m/z = 300.3 (M + H)⁺, theoretical m/z = 299.4.

### [Example 8-3] Synthesis of N-{3-[(1-{2-[(tert-butyldiphenylsilyl)oxy]acetyl}-3-[4-(methoxymethyl)-1,2-dicobaltatricyclo[1.1.0.0^{2,4}]butan-3-yl]azetidin-3-yl)oxy]propyl}methanesulfonamide; hexakis(methanidylidyneoxidanium)

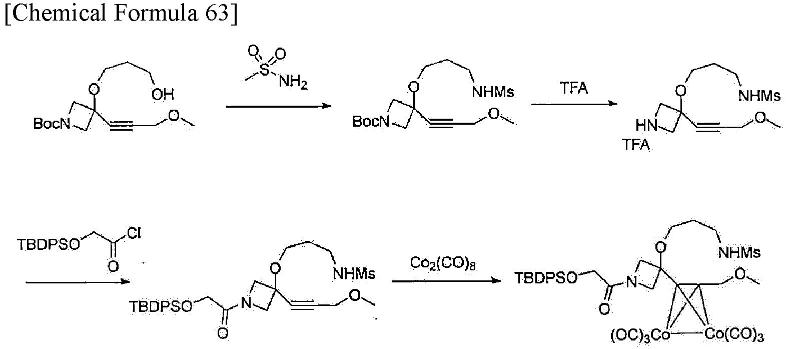

The tert-butyl 3-(3-hydroxypropoxy)-3-(3-methoxyprop-1-yn-1-yl)azetidine-1-carboxylate obtained in Example 8-2 (3.00 g, 10.0 mmol) was dissolved in toluene (33.4 mL), and MsNH₂ (2.38 g, 25.1 mmol) and cyanomethylenetributylphosphorane (6.57 mL, 25.1 mmol, CAS: 157141-27-0) were added. The mixture was stirred at 80°C for 2 hours. After completion of the reaction, the mixture was concentrated under reduced pressure. The obtained residue was purified by flash column chromatography using Isolera (Biotage; Sfaer HC Duo; mobile phase: a gradient from 30% to 100% EtOAc in normal hexane) to obtain an intermediate as a red viscous substance. The intermediate was dissolved in DCM (50 mL), and TFA (15.3 mL, 200 mmol) was added. The resulting reactionmixture was stirred at room temperature for 1.5 hours. After completion of the reaction, the reaction mixture was concentrated under reduced pressure and azeotroped with toluene. The obtained residue was dissolved in a mixed solvent of 1,4-dioxane (20 mL) and water (20 mL), and sodium carbonate (5.31 g, 50.1 mmol) was added at 0°C. Subsequently, a separately prepared solution of 2-[[(1,1-dimethylethyl)diphenylsilyl]oxy]acetyl chloride (4.66 g, 14.0 mmol, CAS: 93853-60-2) in 1,4-dioxane was added dropwise at 0°C, followed by stirring at room temperature for 1 hour. The reaction was quenched by adding brine. Subsequently, the aqueous layer was extracted with EtOAc, and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 100% EtOAc in normal heptane) to obtain an intermediate as a brown viscous substance. After the intermediate thus obtained was dissolved in DCM (100 mL), Co₂(CO)₈ (3.77 g, 11.02 mmol) was added at room temperature. The mixture was stirred for 2 hours. After completion of the reaction, the mixture was concentrated under reduced pressure. The obtained residue was purified by flash column chromatography using Isolera (Biotage; Sfaer HC Duo; mobile phase: a gradient from 20% to 50% EtOAc in normal hexane) to obtain the title compound (5.20 g, 6.06 mmol) as a brown viscous substance.

Analysis condition A: Retention time: 2.36 min; ESI-MS (+); found m/z = 859.2 (M + H)⁺, theoretical m/z = 858.1.

### [Example 8-4] Synthesis of 2-[(tert-butyldiphenylsilyl)oxy]-1- { 7'-methanesulfonyl-3'-oxa-7'-aza-10',11'-dicobaltaspiro[azetidine-3,2'-tetracyclo[7.2.0.0^{1,10}.0^{9,11}]undecan]-1-yl}ethan-1-one; hexakis(methanidylidyneoxidanium)

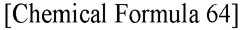

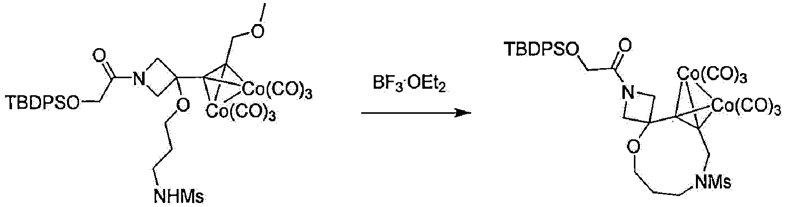

The N-{3-[(1-{2-[(tert-butyldiphenylsilyl)oxy]acetyl}-3-[4-(methoxymethyl)-1,2-dicobaltatricyclo[1.1.0.0^{2,4}]butan-3-yl]azetidin-3-yl)oxy]propyl}methanesulfonamide; hexakis(methanidylidyneoxidanium) obtained in Example 8-3 (4.50 g, 5.24 mmol) was dissolved in DCM (105 mL) and a boron trifluoride-diethyl ether complex (1.99 mL, 15.7 mmol) was added at 0°C and the resulting reaction mixture was stirred for 3 hours. The reaction was quenched by adding sodium bicarbonate solution to the reaction mixture and the aqueous layer was extracted with EtOAc. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 50% EtOAc in normal heptane) to obtain the title compound (2.88 g, 3.48 mmol) as a reddish brown viscous substance.

Analysis condition A: Retention time: 2.34 min; ESI-MS (+); found m/z = 827.3 (M + H)⁺, theoretical m/z = 826.1.

### [Example 8-5] Synthesis of 2-hydroxy-1-{9-methanesulfonyl-5-oxa-2,9-diazaspiro[3.8]dodec-11-yn-2-yl}ethan-1-one

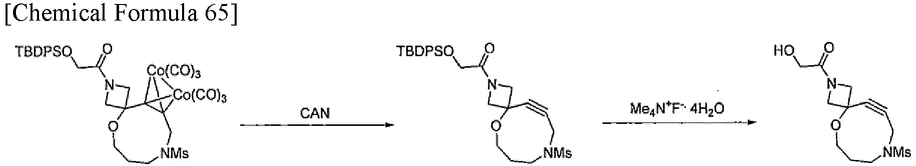

The 2-[(tert-butyldiphenylsilyl)oxy]-1-{7'-methanesulfonyl-3'-oxa-7'-aza-10',11'-dicobaltaspiro[azetidine-3,2'-tetracyclo[7.2.0.0^{1,10}.0^{9,11}]undecan]-1-yl}ethan-1-one; hexakis(methanidylidyneoxidanium) obtained in Example 8-4 (2.5 g, 3.0 mmol) was dissolved in THF (101 mL), followed by the addition of CAN (9.95 g, 18.1 mmol) at 0°C. After stirring at 0°C for 3 hours, the reaction mixture was added dropwise to a mixed solvent, cooled to 0°C, of EtOAc (50 mL) and a saturated aqueous sodium bicarbonate solution (50 mL) to quench the reaction. The aqueous layer was extracted with EtOAc and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 80% EtOAc in normal hexane) to obtain a colorless solid compound. The obtained compound (1.0 g, 1.85 mmol) was dissolved in THF (18.5 mL), and then, acetic acid (0.85 mL, 14.8 mmol) was added at 0°C. Furthermore, tetramethylammonium fluoride tetrahydrate (1.38 g, 14.8 mmol, CAS: 17787-40-5) was added to the reaction mixture and stirred at room temperature for 18 hours. After the reaction mixture was concentrated under reduced pressure, the residue was washed with MeOH, and the white solid was filtered and dried to obtain the title compound (391 mg, 1.29 mmol).

Analysis condition A: Retention time: 1.25 min; ESI-MS (+); found m/z = 303.1 (M + H)⁺, theoretical m/z = 302.4.

¹H-NMR (500 MHz, CDCl₃) δ 4.25-4.10 (m, 4H), 4.05-3.95 (m, 4H), 3.94-3.88 (m, 2H), 3.46-3.40 (m, 2H), 3.02-2.96 (m, 1H), 2.86 (s, 3H), 1.96-1.88 (m, 2H).

### [Example 8-6] Synthesis of 1-{[(2-{9-methanesulfonyl-5-oxa-2,9-diazaspiro[3.8]dodec-11-yn-2-yl}-2-oxoethoxy)carbonyl]oxy}pyrrolidine-2,5-dione

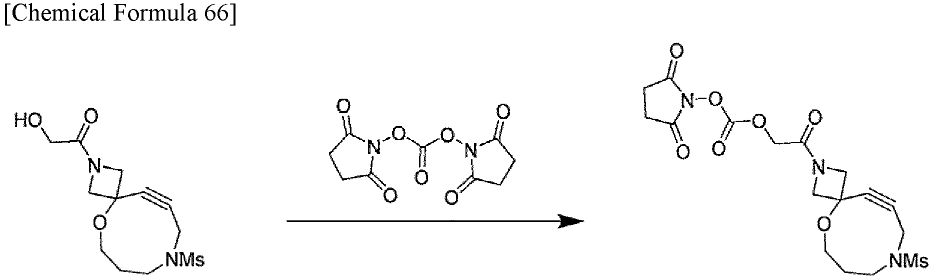

The 2-hydroxy-1-{9-methanesulfonyl-5-oxa-2,9-diazaspiro[3.8]dodec-11-yn-2-yl}ethan-1-one obtained in Example 8-5 (151 mg, 0.50 mmol) was dissolved in acetonitrile (10 mL), and N,N-diisopropylethylamine (262 µL, 1.50 mmol) and bis(2,5-dioxopyrrolidin-1-yl) carbonate (384 mg, 1.50 mmol, CAS: 74124-79-1) was added at room temperature. The resulting reaction mixture was warmed to 40°C and stirred for 1 hour. After the reaction mixture was concentrated under reduced pressure, it was diluted with EtOAc and water and was extracted with EtOAc. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 100% EtOAc in normal hexane) to obtain the title compound (130 mg, 0.29 mmol) as a white solid.

Analysis condition A: Retention time: 1.86 min; ESI-MS (+); found m/z = 444.2 (M + H)⁺, theoretical m/z = 443.4.

¹H-NMR (500 MHz, CDCl₃) δ 4.80 (s, 2H), 4.49-4.38 (m, 2H), 4.23-4.14 (m, 2H), 4.05-3.87 (m, 4H), 3.46-3.38 (m, 2H), 2.89-2.83 (m, 7H), 1.96-1.88 (m, 2H).

### [Example 9-1] Synthesis of tert-butyl 3-{[(4-hydroxybut-2-yn-1-yl)oxy]methyl}-3-(hydroxymethyl)azetidine-1-carboxylate

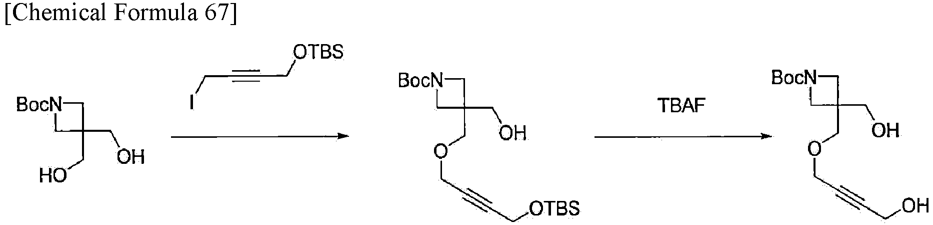

The tert-butyl 3,3-bis(hydroxymethyl)azetidine-1-carboxylate obtained by the method of Example 1-1 (8.11 g, 37.3 mmol) was dissolved in THF (93 mL)/DMF (93 mL) and cooled to 0°C, and then LHMDS (28.7 mL, 37.3 mmol) was added and stirred at 0°C for 30 minutes. (1,1-Dimethylethyl)[(4-iodo-2-butin-1-yl)oxy]dimethylsilane (11.8 g, 38.0 mmol, CAS: 219996-95-9) was added to the reaction mixture and stirred at 0°C for 3 hours. The reaction was quenched by adding a saturated aqueous ammonium chloride solution. Subsequently, the aqueous layer was extracted with EtOAc, and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 0% to 50% EtOAc in normal heptane) to obtain an intermediate as a brown viscous substance. After the obtained intermediate was dissolved in THF (55.4 mL), 1 M TBAF (54.8 mL, 54.8 mmol) was added at room temperature and stirred for 1 hour. The reaction was quenched by adding water. Subsequently, the aqueous layer was extracted with dichloroethane and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 80% to 100% EtOAc in normal heptane, followed by 15% MeOH in DCM) to obtain the title compound (5.0 g, 17.5 mmol) as a light brown viscous substance.

Analysis condition A: Retention time: 1.24 min; ESI-MS (+); found m/z = 286.2 (M + H)⁺, theoretical m/z = 285.2.

### [Example 9-2] Synthesis of hexakis(methanidylidyneoxidanium); tert-butyl 3',7'-dioxa-10',11'-dicobaltaspiro[azetidine-3,5-tetracyclo[7.2.0.0^{1,10}.0^{9,11}]undecane]-1-carboxylate

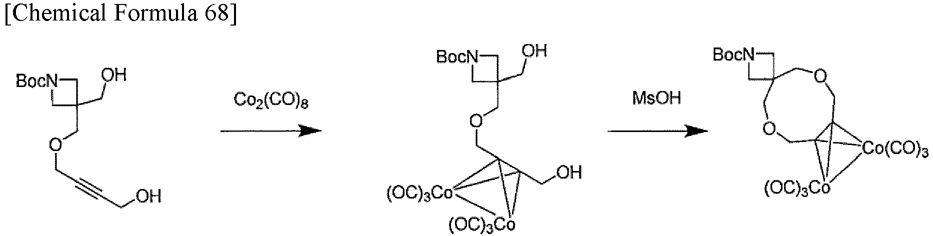

The tert-butyl 3-{[(4-hydroxybut-2-yn-1-yl)oxy]methyl}-3-(hydroxymethyl)azetidine-1-carboxylate obtained in Example 9-1 (1.00 g, 3.50 mmol) was dissolved in DCM (35 mL) and Co₂(CO)₈ (1.44 g, 4.21 mmol) was added at room temperature. The mixture was stirred for 1.5 hours. After completion of the reaction, the mixture was concentrated under reduced pressure. The obtained residue was purified by flash column chromatography using Isolera (Biotage; Sfaer HC Duo; mobile phase: a gradient from 50% to 80% EtOAc in normal heptane) to obtain an intermediate as a brown viscous substance. The above obtained intermediate was dissolved in DCM (292 mL) and methanesulfonic acid (0.076 mg, 0.788 mmol, CAS: 75-75-2) was added at room temperature. The mixture was stirred for 3 hours. The reaction was quenched by adding a saturated aqueous sodium bicarbonate solution to the reaction mixture and the aqueous layer was extracted with DCM. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 30% EtOAc in normal heptane) to obtain the title compound (0.124 g, 0.224 mmol) as a brown viscous substance.

Analysis condition A: Retention time: 2.08 min; ESI-MS (+); found m/z = 498.0 (M - C₄H₈ + H)⁺, theoretical m/z = 553.0.

¹H-NMR (500 MHz, CDCl₃) δ 5.03 (s, 4H), 3.99 (s, 4H), 3.64 (s, 4H), 1.43 (s, 9H).

### [Example 9-3] Synthesis of tert-butyl 6,11-dioxa-2-azaspiro[3.8]dodec-8-yn-2-carboxylate1

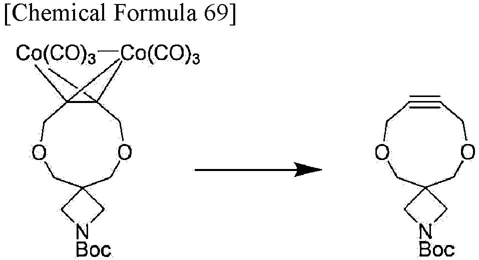

The hexakis(methanidylidyneoxidanium); tert-butyl 3',7'-dioxa-10',11'-dicobaltaspiro[azetidine-3,5'-tetracyclo[7.2.0.0^{1,10}.0^{9,11}]undecane]-1-carboxylate obtained in Example 9-2 (0.124 g, 0.224 mmol) was dissolved in THF (22 mL), and then, CAN (0.737 g, 1.35 mmol) was added at 0°C. After stirring at 0°C for 1 hour, the reaction mixture was added dropwise to a mixed solvent, cooled to 0°C, of EtOAc (200 mL) and a saturated aqueous sodium bicarbonate solution (100 mL) to quench the reaction. The aqueous layer was extracted with EtOAc and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 30% EtOAc in normal heptane) to obtain the title compound (0.026 g, 0.097 mmol) as colorless powder.

Analysis condition A: Retention time: 1.49 min; ESI-MS (+); found m/z = 212.1 (M - C₄H₈ + H)⁺, theoretical m/z = 267.2.

¹H-NMR (500 MHz, CDCl₃) δ 4.16 (s, 4H), 3.99 (s, 4H), 3.59 (s, 4H), 1.43 (s, 9H).

### [Example 9-4] Synthesis of 2-[(tert-butyldiphenylsilyl)oxy]-1-{6,11-dioxa-2-azaspiro[3.8]dodec-8-yn-2-yl}ethan-1-one

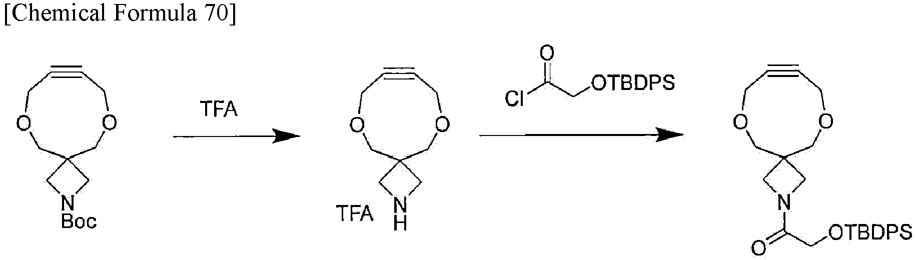

The tert-butyl 6,11-dioxa-2-azaspiro[3.8]dodec-8-yn-2-carboxylate obtained in Example 9-3 (0.026 g, 0.097 mmol) was dissolved in DCM (1.0 mL), and then, TFA (0.225 mL, 2.92 mmol) was added at room temperature. After stirring at room temperature for 1 hour, toluene was added. Then, the mixture was concentrated under reduced pressure and further azeotroped with toluene. After 1,4-dioxane (1.0 mL)/water (1.0 mL) was added to the obtained residue, sodium carbonate (103 mg, 0.97 mmol) was added at 0°C. Furthermore, a separately prepared solution of 2-[[(1,1-dimethylethyl)diphenylsilyl]oxy]acetyl chloride (0.080 mg, 0.243 mmol, CAS: 93853-60-2) in 1,4-dioxane was added dropwise at 0°C, followed by stirring at room temperature for 1 hour. After completion of the reaction, the reaction was quenched by adding a saturated aqueous sodium bicarbonate solution. The aqueous layer was extracted with EtOAc and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 50% EtOAC in normal heptane) to obtain the title compound (0.041 g, 0.088 mmol) as a colorless viscous substance.

Analysis condition A: Retention time: 2.04 min; ESI-MS (+); found m/z = 464.3 (M + H)⁺, theoretical m/z = 463.6.

¹H-NMR (500 MHz, CDCl₃) δ 7.67-7.61 (m, 4H), 7.48-7.37 (m, 6H), 4.21-4.12 (m, 6H), 4.02-3.90 (m, 6H), 3.68 (s, 2H), 1.07 (s, 9H).

### [Example 9-5] Synthesis of 1-{6,11-dioxa-2-azaspiro[3.8]dodec-8-yn-2-yl}-2-hydroxyethan-1-one

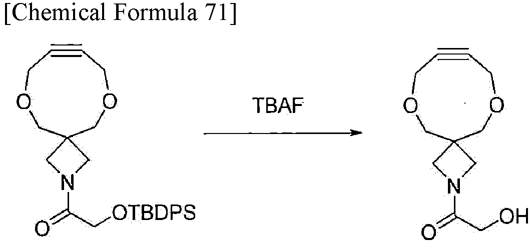

The 2-[(tert-butyldiphenylsilyl)oxy]-1-{6,11-dioxa-2-azaspiro[3.8]dodec-8-yn-2-yl}ethan-1-one obtained in Example 9-4 (0.041 g, 0.088 mmol) was dissolved in THF (1.0 mL), and then, TBFA (0.132 mL, 0.13 mmol, 1M THF solution) was added at room temperature. After stirring for 1.5 hours at room temperature, the reaction mixture was concentrated under reduced pressure. The obtained residue was purified by flash column chromatography using Isolera (Biotage; Sfaer HC Duo; mobile phase: a gradient from 50% to 100% EtOAc in normal heptane, followed by a gradient of 5% MeOH in EtOAc) to obtain the title compound (0.018 g, 0.080 mmol) as colorless powder.

Analysis condition A: Retention time: 0.85 min; ESI-MS (+); found m/z = 226.2 (M + H)⁺, theoretical m/z = 225.2.

¹H-NMR (500 MHz, CDCl₃) δ 4.17 (s, 4H), 4.06-3.96 (m, 4H), 3.97 (s, 2H), 3.82-3.61 (m, 4H), 3.06 (s, 1H).

### [Example 10-1] Synthesis of tert-butyl 4,4-bis(hydroxymethyl)piperidine-1-carboxylate

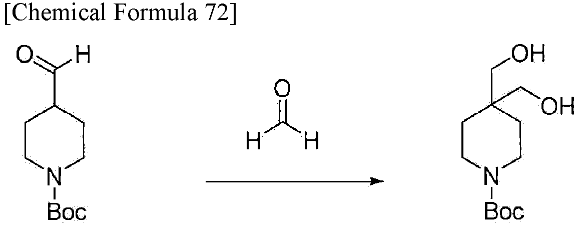

tert-Butyl 4-formylpiperidine-1-carboxylate (10.0 g, 46.9 mmol, CAS: 137076-22-3) was dissolved in MeOH (150 mL). To this, H₂O (50 mL) in which KOH (10.92 g, 195 mmol) was dissolved and a 37% aqueous formaldehyde solution (38.4 mL, 516 mmol) were added. The resulting reaction mixture was stirred at room temperature for 150 minutes and then at 40°C for 5 hours. MeOH was distilled away from the reaction mixture under reduced pressure. A saturated aqueous ammonium chloride solution was added and extracted with EtOAc. The obtained organic layer was dried over sodium sulfate, then filtered, and concentrated under reduced pressure. The obtained residue was purified by flash column chromatography using Isolera (Biotage; Sfaer HC Duo; mobile phase: a gradient from 3% to 15% MeOH in DCM). Subsequently, the fraction containing the target substance was concentrated under reduced pressure to obtain the title compound (10.0 g, 40.8 mmol) as colorless powder.

¹H-NMR (500 MHz, CDCl₃) δ 3.67 (s, 4H), 3.44-3.36 (m, 4H), 2.39 (s, 2H), 1.49-1.44 (m, 13H).

### [Example 10-2] Synthesis of (9H-fluoren-9-yl)methyl 4,4-bis(hydroxymethyl)piperidine-1-carboxylate

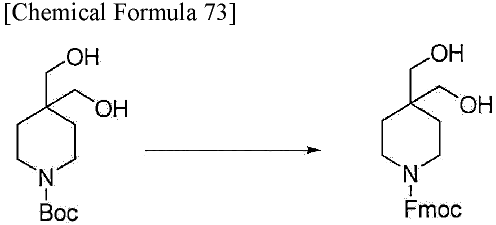

The tert-butyl 4,4-bis(hydroxymethyl)piperidine-1-carboxylate obtained in Example 10-1 (1.00 g, 4.08 mmol) was dissolved in 1,4-dioxane (10.2 mL) and a solution of hydrogen chloride in 1,4-dioxane (4M, 10.2 mL, 40.8 mmol) was added. The resulting reaction mixture was stirred at room temperature for 5 hours, and then concentrated under reduced pressure. The obtained residue and DIPEA (1.05 g, 8.15 mmol) were dissolved in DCM (20 mL), and Fmoc-OSu (1.25 g, 3.87 mmol) was added at room temperature. The reaction mixture as obtained was stirred for 2 hours. DCM was added to the reaction mixture for extraction, and the obtained organic layer was concentrated under reduced pressure. The obtained residue was purified by flash column chromatography using Isolera (Biotage; Sfaer HC Duo; mobile phase: a gradient from 0% to 15% MeOH in DCM). Subsequently, the fraction containing the target substance was concentrated under reduced pressure to obtain the title compound (1.45 g, 3.95 mmol) as a colorless viscous substance.

Analysis condition A: Retention time: 1.56 min; ESI-MS (+); found m/z = 368.2 (M + H)⁺, theoretical m/z = 367.2.

### [Example 10-3] Synthesis of hexakis(methanidylidyneoxidanium); (9H-fluoren-9-yl)methyl 3',7'-dioxa-10',11'-dicobaltaspiro[piperidine-4,5'-tetracyclo[7.2.0.0^{1,10}.0^{9,11}]undecane]-1-carboxylate

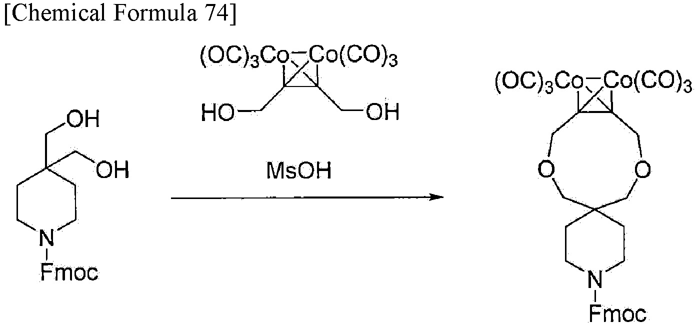

The (9H-fluoren-9-yl)methyl 4,4-bis(hydroxymethyl)piperidine-1-carboxylate obtained in Example 10-2 (250 mg, 0.68 mmol) and methanesulfonic acid (0.131 g, 1.36 mmol) were dissolved in DCM (54.4 mL). To this solution, a solution of hexakis(methanidylidyneoxidanium); [4-(hydroxymethyl)-1,2-dicobaltatricyclo[1.1.0.0^{2,4}]butan-3-yl]MeOH (253 mg, 0.68 mmol, CAS: 55975-76-3, synthesized by the method described in Organic Letters (2015), 17(16), 4086-4089) in DCM (13.6 mL) was added dropwise at room temperature over a period of 90 minutes. Furthermore, after stirring at room temperature for 30 minutes, the reaction was quenched by adding a 10% aqueous sodium bicarbonate solution, followed by the extraction with DCM. The obtained organic layer was concentrated under reduced pressure. The obtained residue was purified by flash column chromatography using Isolera (Biotage; Sfaer HC Duo; mobile phase: a gradient from 10% to 30% EtOAc in normal heptane). Subsequently, the fraction containing the target substance was concentrated under reduced pressure to obtain the title compound (280 mg, 0.398 mmol) as brown powder.

Analysis condition A: Retention time: 2.22 min; ESI-MS (+); found m/z = 704.2 (M + H)⁺, theoretical m/z = 703.0.

### [Example 10-4] Synthesis of (9H-fluoren-9-yl)methyl 8,13-dioxa-3-azaspiro[5.8]tetradec-10-yne-3-carboxylate

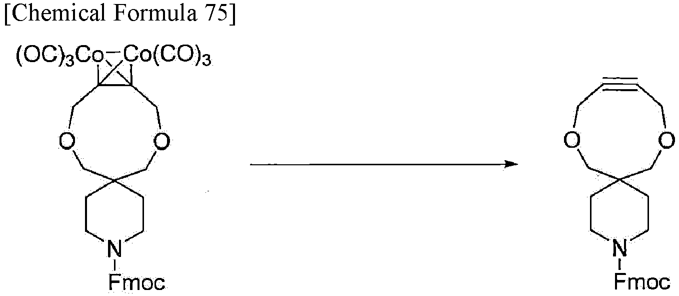

The hexakis(methanidylidyneoxidanium); (9H-fluoren-9-yl)methyl 3',7'-dioxa-10',11'-dicobaltaspiro[piperidine-4,5'-tetracyclo[7.2.0.0^{1,10}.0^{9,11}]undecane]-1-carboxylate obtained in Example 10-3 (0.100 g, 0.142 mmol) was dissolved in THF (14.2 mL), and then, CAN (0.234 g, 0.426 mmol) was added at 0°C. After stirring at 0°C for 2 hours, Et₂O and a 10% aqueous sodium bicarbonate solution were added to quench the reaction. The aqueous layer was extracted with Et₂O and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 40% EtOAc in normal heptane) to obtain the title compound (0.025 g, 0.060 mmol) as a colorless viscous substance.

Analysis condition A: Retention time: 1.87 min; ESI-MS (+); found m/z = 418.3 (M + H)⁺, theoretical m/z = 417.2.

¹H-NMR (500 MHz, CDCl₃) δ 7.76 (d, J=7.5Hz, 2H), 7.57 (d, J=7.5Hz, 2H), 7.43-7.36 (m, 2H), 7.34-7.28 (m, 2H), 4.42 (d, J=6.9Hz, 2H), 4.24 (t, J=6.9Hz, 1H), 4.20-4.05 (m, 4H), 3.91-3.71 (m, 2H), 3.67-3.54 (m, 2H), 3.51-3.37 (m, 4H), 1.57-1.39 (m, 2H), 1.36-1.16 (m, 2H).

### [Example 11-1] Synthesis of tert-butyl 4-{[(tert-butyldimethylsilyl)oxy]methyl}-4-(hydroxymethyl)piperidine-1-carboxylate

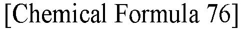

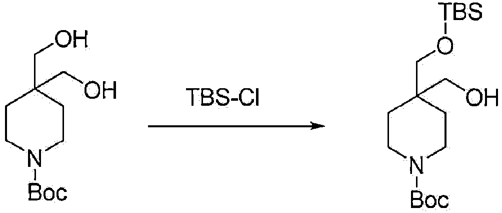

The tert-butyl 4,4-bis(hydroxymethyl)piperidine-1-carboxylate obtained in Example 10-1 (1.00 g, 4.08 mmol) was dissolved in THF (40.8 mL), and sodium hydride (163 mg, 4.08 mmol, 60%, dispersed in liquid paraffin) was added at 0°C under ice-cooling. The resulting reaction mixture was allowed to warm to room temperature and stirred for 30 minutes. Tertiary-butyldimethylsilyl chloride (614 mg, 4.08 mmol, CAS: 18162-48-6) was added to the reaction mixture and stirred at room temperature overnight. Under ice-cooling, a saturated aqueous ammonium chloride solution was added to the reaction mixture and EtOAc was added to perform extraction. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 50% EtOAc in normal heptane). Subsequently, the fraction containing the target substance was concentrated under reduced pressure to obtain the title compound (1.45 g, 4.03 mmol) as colorless powder.

Analysis condition A: Retention time: 2.00 min; ESI-MS (+); found m/z = 360.3 (M + H)⁺, theoretical m/z = 359.3.

### [Example 11-2] Synthesis of tert-butyl 4-[({4-[(tert-butyldimethylsilyl)oxy]but-2-yn-1-yl}oxy)methyl]-4-{[(tert-butyldimethylsilyl)oxy]methyl}piperidine-1-carboxylate

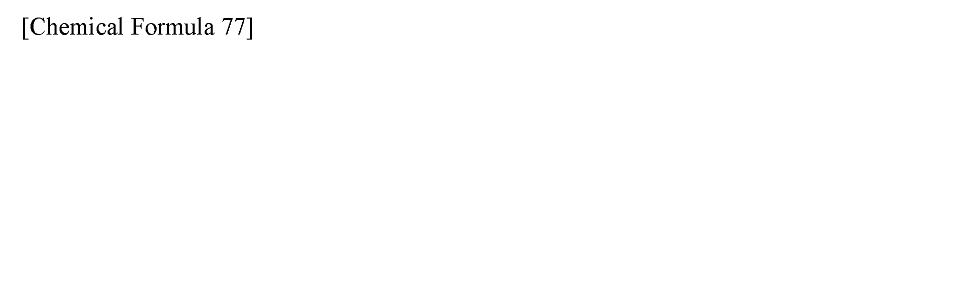

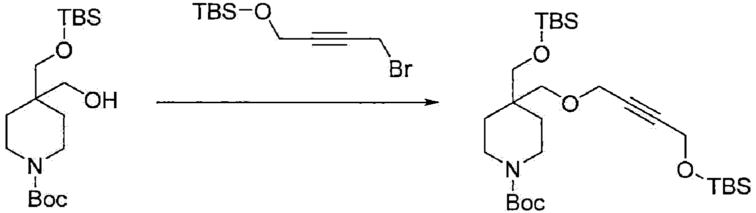

The tert-butyl 4-{[(tert-butyldimethylsilyl)oxy]methyl}-4-(hydroxymethyl)piperidine-1-carboxylate obtained in Example 11-1 (0.50 g, 1.39 mmol) was dissolved in a mixed solvent of THF (11.1 mL) and DMF (2.78 mL), and sodium hydride (55.6 mg, 1.39 mmol, 60%, dispersed in liquid paraffin) was added under ice-cooling. The resulting reaction mixture was allowed to warm to room temperature and stirred for 10 minutes. [(4-Bromobut-2-yn-1-yl)oxy](tert-butyl)dimethylsilane (732 mg, 2.78 mmol, CAS: 110796-98-0) was added to the reaction mixture and stirred at room temperature for 7 hours. A saturated aqueous ammonium chloride solution was added to the reaction mixture and EtOAc was added to perform extraction. The obtained organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 40% EtOAc in normal heptane). Subsequently, the fraction containing the target substance was concentrated under reduced pressure to obtain the title compound (0.192 g, 0.354 mmol) as a yellow oily substance.

Analysis condition A: Retention time: 2.71 min; ESI-MS (+); found m/z = 542.4 (M + H)⁺, theoretical m/z = 541.4.

### [Example 11-3] Synthesis of tert-butyl 4-{[(4-hydroxybut-2-yn-1-yl)oxy]methyl}-4-(hydroxymethyl)piperidine-1-carboxylate

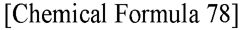

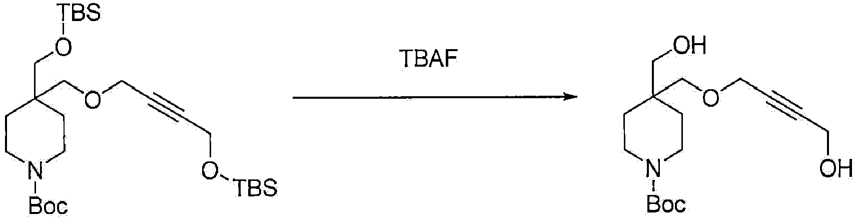

The tert-butyl 4-[({4-[(tert-butyldimethylsilyl)oxy]but-2-yn-1-yl}oxy)methyl]-4-{[(tert-butyldimethylsilyl)oxy]methyl}piperidine-1-carboxylate obtained in Example 11-2 (0.195 g, 0.360 mmol) was dissolved in THF (3.6 mL) and 1 M TBAF (0.792 mL, 0.792 mmol, THF solution) was added at room temperature. After stirring at room temperature for 2 hours, the reaction mixture was concentrated under reduced pressure. The obtained residue was purified by flash column chromatography using Isolera (Biotage; Sfaer HC Duo; mobile phase: a gradient from 10% to 80% EtOAc in normal heptane) to obtain the title compound (0.102 g, 0.325 mmol) as a light yellow oily substance.

Analysis condition A: Retention time: 1.40 min; ESI-MS (+); found m/z = 314.3 (M + H)⁺, theoretical m/z = 313.2.

### [Example 11-4] Synthesis of hexakis(methanidylidyneoxidanium); tert-butyl 4-(hydroxymethyl)-4-({[4-(hydroxymethyl)-1,2-dicobaltatricyclo[1.1.0.0^{2,4}]butan-3-yl]methoxy}methyl)piperidine-1-carboxylate

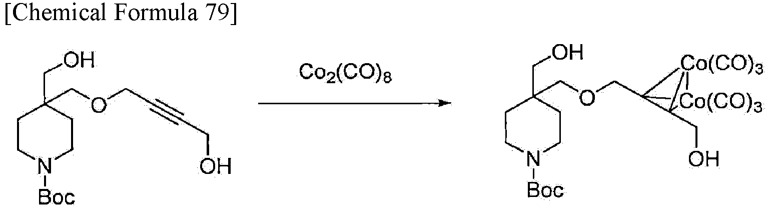

The tert-butyl 4-{[(4-hydroxybut-2-yn-1-yl)oxy]methyl}-4-(hydroxymethyl)piperidine-1-carboxylate obtained in Example 11-3 (0.102 g, 0.325 mmol) was dissolved in DCM (3.3 mL) and Co₂(CO)₈ (0.134 g, 0.391 mmol) was added at room temperature. The mixture was stirred for 2 hours. After completion of the reaction, the mixture was concentrated under reduced pressure. The obtained residue was purified by flash column chromatography using Isolera (Biotage; Sfaer HC Duo; mobile phase: a gradient from 10% to 80% EtOAc in normal heptane) to obtain the title compound (0.170 g, 0.284 mmol) as a brown oily substance.

Analysis condition A: Retention time: 1.96 min; ESI-MS (+); found m/z = 582.1 (M - H₂O + H)⁺, theoretical m/z = 599.0.

### [Example 11-5] Synthesis of hexakis(methanidylidyneoxidanium); tert-butyl 3',7'-dioxa-10',11'-dicobaltaspiro[piperidine-4,5'-tetracyclo[7.2.0.0^{1,10}.0^{9,11}]undecane]-1-carboxylate

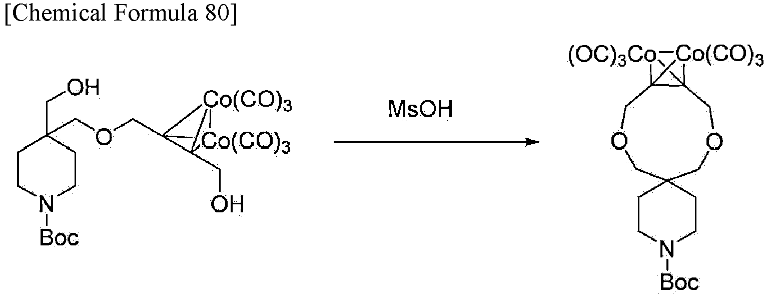

The hexakis(methanidylidyneoxidanium); tert-butyl 4-(hydroxymethyl)-4-({[4-(hydroxymethyl)-1,2-dicobaltatricyclo[1.1.0.0^{2,4}]butan-3-yl]methoxy}methyl)piperidine-1-carboxylate obtained in Example 11-4 (0.138 g, 0.230 mmol) was dissolved in DCM (7.7 mL) and methanesulfonic acid (0.020 g, 0.207 mmol) was added at room temperature. The resulting reaction mixture was stirred for 3 hours. Triethylamine (0.117 g, 1.15 mmol, CAS: 121-44-8) and ditertiary butyl dicarbonate (0.101 g, 0.461 mmol, CAS: 24424-99-5) were added to the reaction mixture and stirred at room temperature for 20 minutes. The reaction mixture was purified by flash column chromatography using Isolera (Biotage; Sfaer HC Duo; mobile phase: a gradient from 5% to 80% EtOAc in normal heptane) to obtain the title compound (0.055 g, 0.095 mmol) as brown powder.

Analysis condition A: Retention time: 2.19 min; ESI-MS (+); found m/z = 526.0 (M - C₄H₈ + H)⁺, theoretical m/z = 581.0.

### [Example 11-6] Synthesis of tert-butyl 8,13-dioxa-3-azaspiro[5.8]tetradec-10-yne-3-carboxylate

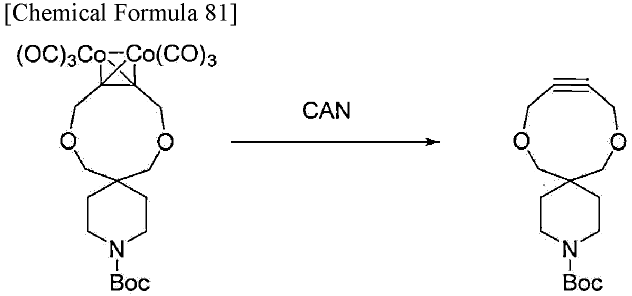

The hexakis(methanidylidyneoxidanium); tert-butyl 3',7'-dioxa-10',11'-dicobaltaspiro[piperidine-4,5'-tetracyclo[7.2.0.0^{1,10}.0^{9,11}]undecane]-1-carboxylate obtained in Example 11-5 (0.060 g, 0.103 mmol) was dissolved in THF (10.0 mL), and then, CAN (0.340 g, 0.619 mmol) was added at 0°C. After stirring at 0°C for 1 hour, the reaction mixture was added dropwise to a mixed solvent, cooled to 0°C, of EtOAc and a 10% aqueous sodium bicarbonate solution to stop the reaction. The aqueous layer was extracted with EtOAc and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 40% EtOAc in normal hexane) to obtain the title compound (0.017 g, 0.057 mmol) as white powder.

Analysis condition A: Retention time: 1.69 min; ESI-MS (+); found m/z = 240.1 (M - C₄H₈ + H)⁺, theoretical m/z = 295.2.

¹H-NMR (500 MHz, CDCl₃) δ 4.26-4.03 (m, 4H), 3.80 (s, 2H), 3.60 (s, 2H), 3.39 (s, 4H), 1.58-1.12 (m, 13H).

### [Example 11-7] Synthesis of 8,13-dioxa-3-azaspiro[5.8]tetradec-10-yne hydrochloride

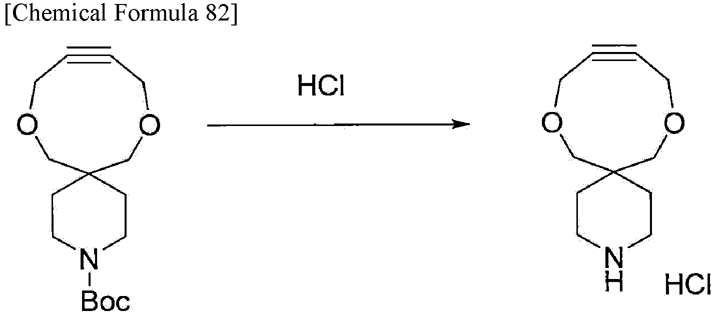

The tert-butyl 8,13-dioxa-3-azaspiro[5.8]tetradec-10-yne-3-carboxylate obtained in Example 11-6 (0.012 g, 0.041 mmol) was dissolved in 1,4-dioxane (0.41 mL), and then, a solution of hydrogen chloride in 1,4-dioxane (4 M, 50.8 µL, 0.203 mmol) was added at room temperature. After stirring for 1 hour at room temperature, a solution of hydrogen chloride in 1,4-dioxane (4M, 85 µL, 0.340 mmol) was added. After stirring for another hour at room temperature, a solution of hydrogen chloride in 1,4-dioxane solution (4M, 270 µL, 1.080 mmol) was added. After stirring for 1 hour at room temperature, the mixture was concentrated and dried under reduced pressure to obtain the title compound as white powder.

¹H-NMR (500 MHz, DMSO-d₆) δ 8.56-8.36 (m, 2H), 4.30-4.03 (m, 4H), 3.75-3.50 (m, 4H), 3.07-2.97 (m, 4H), 1.71-1.18 (m, 4H).

### [Example 12-1] Synthesis of (9H-fluoren-9-yl)methyl 3-(3-hydroxypropoxy)-3-(3-methoxyprop-1-yn-1-yl)azetidine-1-carboxylate

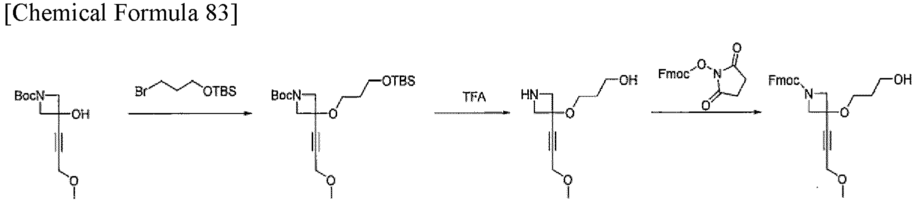

A solution of the tert-butyl 3-hydroxy-3-(3-methoxyprop-1-yn-1-yl)azetidine-1-carboxylate obtained in Example 8-1 (1.20 g, 4.97 mmol) in THF (8.3 mL) was added to DMF (8.3 mL), and sodium hydride (0.298 g, 7.46 mmol, 60%, dispersed in fluid paraffin) was added at 0°C. The mixture was stirred for 20 minutes. Then, a solution of (3-bromopropoxy)(tert-butyl)dimethylsilane (1.89 g, 7.46 mmol, CAS: 89031-84-5) in DMF was added at 0°C and stirred at room temperature for 14 hours. The reaction was quenched by adding an aqueous solution of isopropyl acetate/water (100 mL/100 mL). Subsequently, the aqueous layer was extracted with isopropyl acetate, and the organic layer was washed with brine, then dried over sodium sulfate, then filtered, and concentrated under reduced pressure to obtain an intermediate as colorless liquid. The above obtained intermediate (1.90 g, 4.59 mmol) was dissolved in 11.5 mL of DCM and cooled to 0°C. Then, TFA (11.5 mL, 150 mmol) was added at room temperature and stirred for 2 hours. The reaction mixture was concentrated under reduced pressure. To the obtained residue, potassium bicarbonate (1.43 g, 14.3 mmol), water (10.6 mL), and 1,4-dioxane (10.6 mL) were added and stirred. Subsequently, a solution of (N-9-fluorenylmethoxycarbonyloxy)succinimide (1.45 g, 4.29 mmol) in 1,4-dioxane (10.6 mL) was added dropwise at 0°C. After dropwise addition, the resulting reaction mixture was allowed to warm to room temperature and stirred overnight. Isopropyl acetate (100 mL) and water (100 mL) were added to the reaction mixture. The aqueous layer was extracted with isopropyl acetate, and the organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 20% to 65% EtOAc in normal heptane) to obtain the title compound (264 mg, 0.63 mmol) as a dark brown viscous substance.

Analysis condition A: Retention time: 1.85 min; ESI-MS (+); found m/z = 422.4 (M + H)⁺, theoretical m/z = 421.4.

### [Example 12-2] Synthesis of (9H-fluoren-9-yl)methyl 3-(3-hydroxypropoxy)-3-[4-(methoxymethyl)-1,2-dicobaltatricyclo[1.1.0.0^{2,4}]butan-3-yl]azetidine-1-carboxylate; hexakis(methanidylidyneoxidanium)

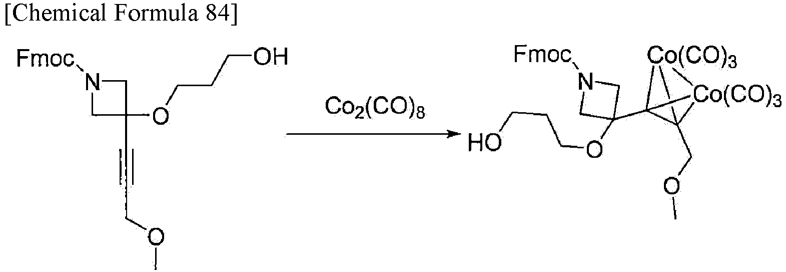

The (9H-fluoren-9-yl)methyl 3-(3-hydroxypropoxy)-3-(3-methoxyprop-1-yn-1-yl)azetidine-1-carboxylate obtained in Example 12-1 (803 mg, 1.91 mmol) was dissolved in DCM (9.5 mL), and Co₂(CO)₈ (782 mg, 2.29 mmol) was added at room temperature. The mixture was stirred for 0.5 hours. After completion of the reaction, the mixture was concentrated under reduced pressure. The obtained residue was purified by flash column chromatography using Isolera (Biotage; Sfaer HC Duo; mobile phase: a gradient from 1% to 15% MeOH in DCM) to obtain an intermediate (1.25 g, 1.77 mmol) as a dark brown viscous substance.

Analysis condition A: Retention time: 2.29 min; ESI-MS (+); found m/z = 676.2 (M - MeOH + H)⁺, theoretical m/z = 707.4.

### [Example 12-3] Synthesis of (9H-fluoren-9-yl)methyl 3'7'-dioxa-10',11'-dicobaltaspiro[azetidine-3,2'-tetracyclo[7.2.0.0^{1,10}.0^{9,11}]undecane]-1-carboxylate; hexakis(methanidylidyneoxidanium)

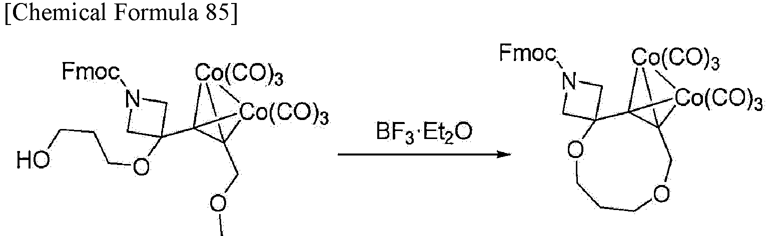

The (9H-fluoren-9-yl)methyl 3-(3-hydroxypropoxy)-3-[4-(methoxymethyl)-1,2-dicobaltatricyclo[1.1.0.0^{2,4}] butan-3-yl]azetidine-1-carboxylate; hexakis(methanidylidyneoxidanium) obtained in Example 12-2 (1.0 g, 1.41 mmol) was dissolved in DCM (283 mL). A boron trifluoride-diethyl ether complex (0.21 mL, 1.70 mmol) was added at 0°C and the resulting reaction mixture was stirred for 2.5 hours. The reaction was quenched by adding a saturated aqueous sodium bicarbonate solution to the reaction mixture and the aqueous layer was extracted with DCM. The organic layer was washed with brine, then dried over sodium sulfate, then filtered, and concentrated under reduced pressure. The obtained residue was purified by flash column chromatography using Isolera (Biotage; Sfaer HC Duo; mobile phase: a gradient from 10% to 60% EtOAc in normal heptane) to obtain the title compound (123 mg, 0.181 mmol) as a dark brown viscous substance.

Analysis condition A: Retention time: 2.38 min; ESI-MS (+); found m/z = 676.2 (M + H)⁺, theoretical m/z = 675.4.

### [Example 12-4] Synthesis of (9H-fluoren-9-yl)methyl 5,9-dioxa-2-azaspiro[3.8]dodec-11-yn-2-carboxylate

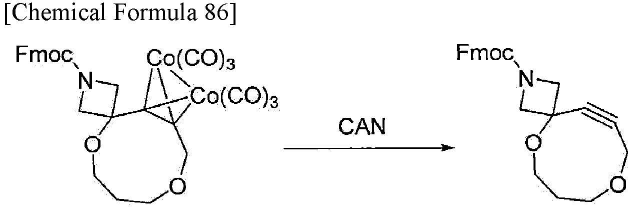

Diisopropyl ether (4.4 mL) and Wakosil (R) c200 (FUJIFILM Wako Pure Chemical Corporation, 2.5 g, CAS: 63231-67-4) was added to the (9H-fluoren-9-yl)methyl 3'7'-dioxa-10',11'-dicobaltaspiro[azetidine-3,2'-tetracyclo[7.2.0.0^{1,10}.0^{9,11}]undecane]-1-carboxylate; hexakis(methanidylidyneoxidanium) obtained in Example 12-3 (150 mg, 0.22 mmol), and then, CAN (1.22 g, 2.22 mmol) was added at 0°C. Then, the resulting reaction mixture was allowed to warm to room temperature and stirred for 3 hours. After completion of the reaction, the reaction mixture was passed through Wakosil (R)-c200 (FUJIFILM Wako Pure Chemical Corporation) to filter out insoluble materials. Subsequently, the filtrate was concentrated under reduced pressure. The obtained residue was purified by flash column chromatography using Isolera (Biotage; Sfaer HC Duo; mobile phase: a gradient from 50% to 80% EtOAc in normal hexane, followed by a gradient from 1% to 20% MeOH in DCM) to obtain the title compound (0.050 g, 0.128 mmol) as light yellow powder.

Analysis condition A: Retention time: 2.03 min; ESI-MS (+); found m/z = 390.3 (M + H)⁺, theoretical m/z = 389.4.

Hereinafter, synthesis examples of conjugates of a peptide and a nucleic acid are provided as examples. The present invention will be described in more detail with reference to these examples, but the present invention is not limited thereto.

For conjugation, the following compounds were used as a peptide, a nucleic acid, and a click reagent.

Peptide compound (CAS: 2699074-52-5. See WO 2021167107. Hereinafter, the peptide may be referred to as "peptide F".)

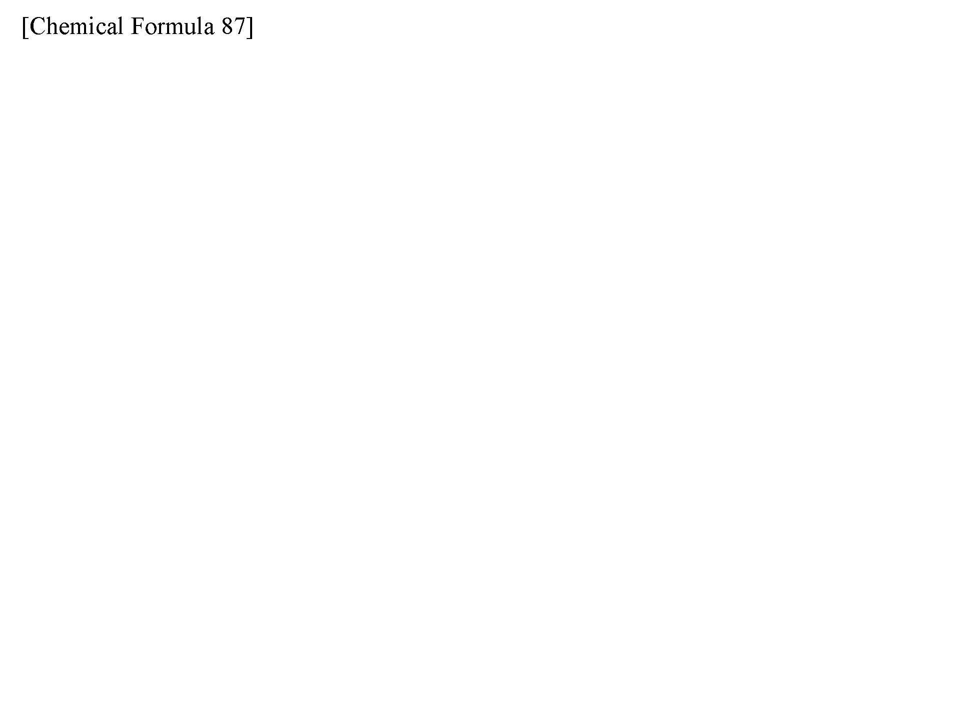

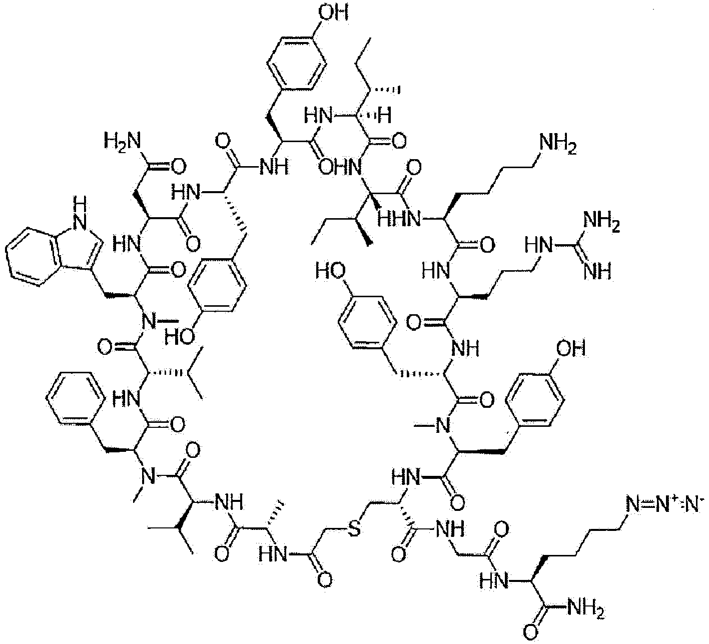

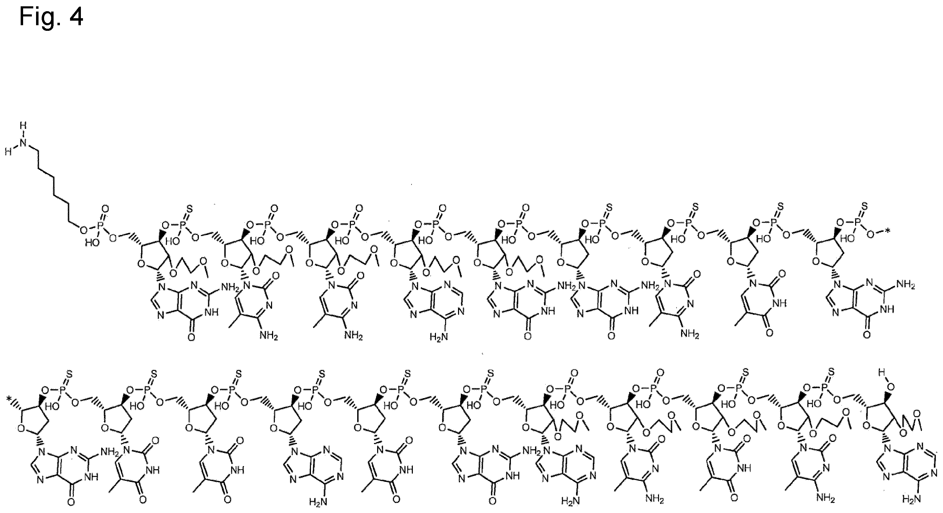

Nucleic acid compound (The nucleic acid compound was synthesized according to a general synthesis method, with reference to US 20180010126. Hereinafter, this compound may be referred to as "NS-S01-20." The structural formula of the nucleic acid compound is shown in Fig. 4.)

The 1-{[(2-{9-methanesulfonyl-5-oxa-2,9-diazaspiro[3.8]dodec-11-yn-2-yl}-2-oxoethoxy)carbonyl]oxy}pyrrolidine-2,5-dione obtained in Example 8-6 is a click reagent compound. (Hereinafter, this compound may be referred to as "click reagent X".)

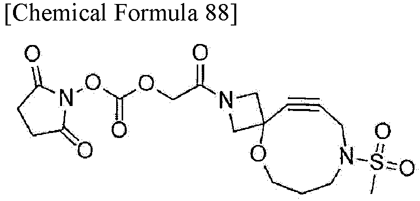

A compound produced by introducing click reagent X into NS-S01-20 is shown below. (Hereinafter, this compound may be referred to as a compound with introduced click reagent X).

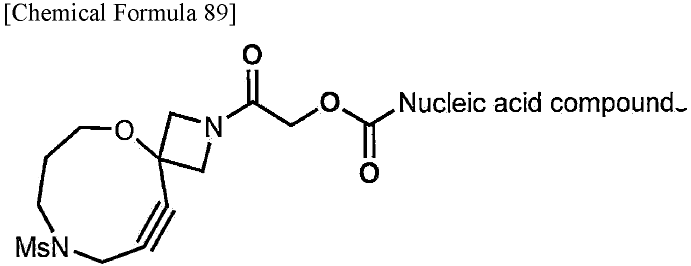

A compound in which a compound with introduced click reagent X and peptide F are conjugated is shown below. (Hereinafter, this compound may be referred to as PN-S01-10).

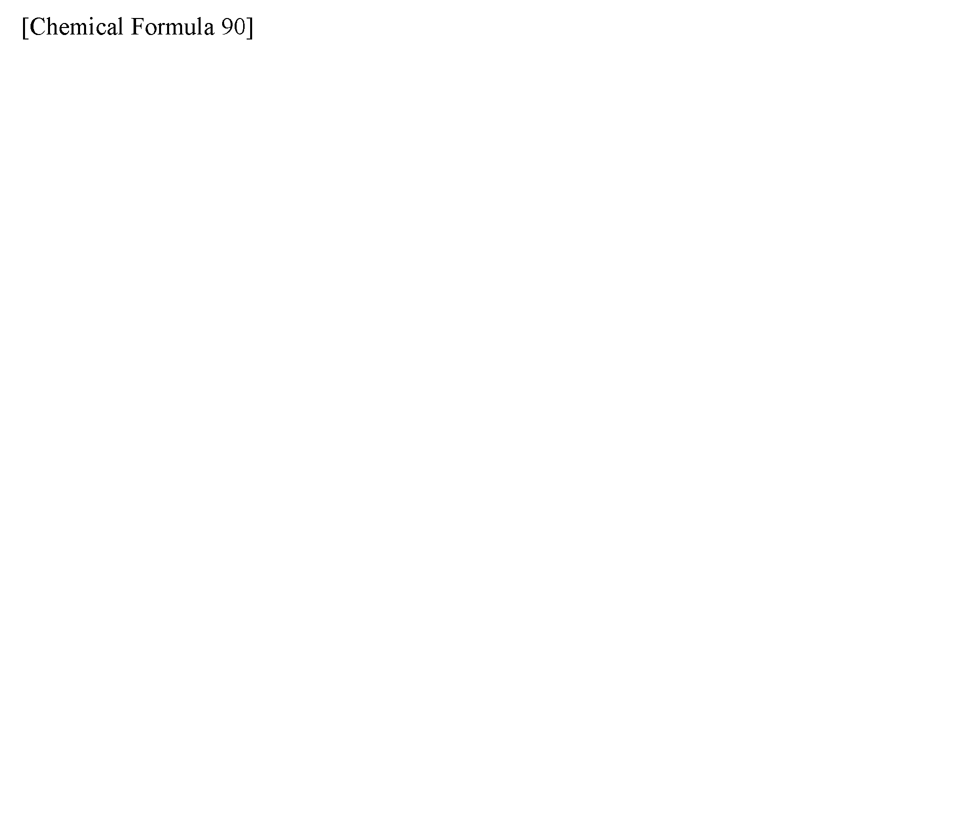

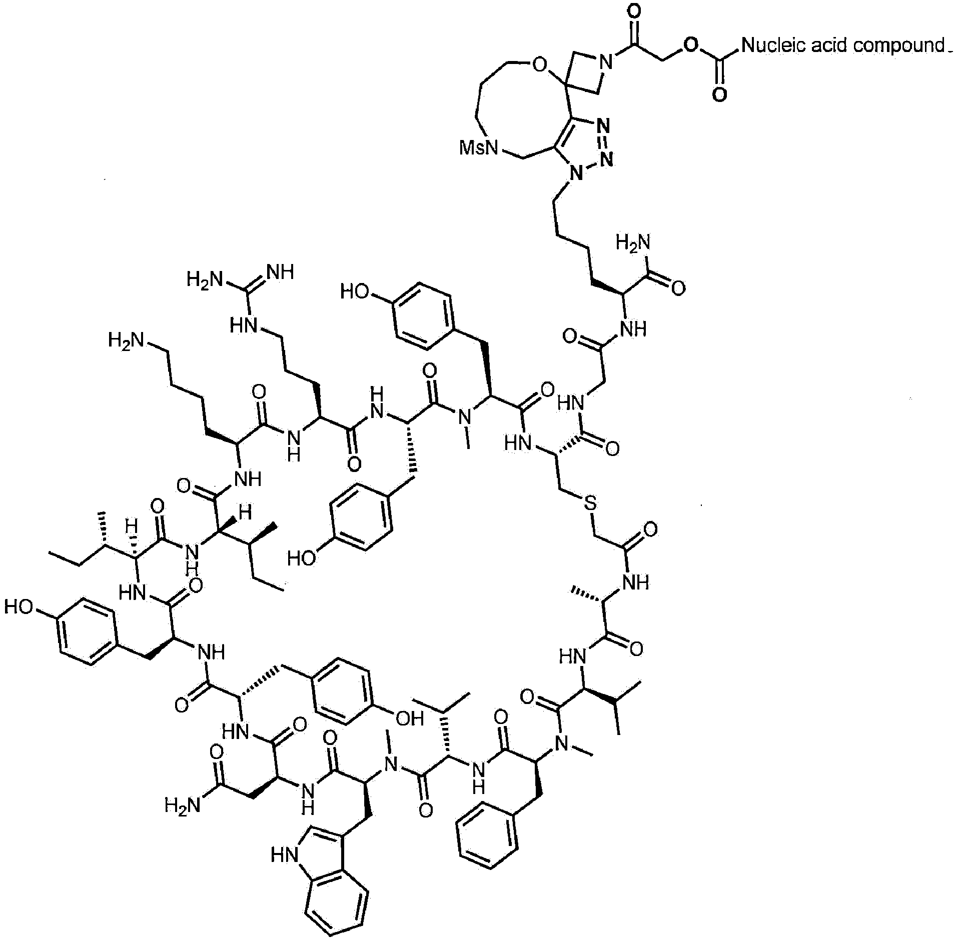

### Analysis condition 1 (LC-MS)

Column: XBridge Premier Oligonucleotide BEH C18 column (2.5 µm), 2.1 mm × 150 mm
(Waters Corporation)
Mobile phase A: 400 mM 1,1,1,3,3,3-hexafluoro-2-propanol, 15 mM solution of triethylamine in water
Mobile phase B: 400 mM 1,1,1,3,3,3-hexafluoro-2-propanol, 15 mM solution of triethylamine in MeOH
Flow rate: 0.2 mL/min
Column temperature: 60°C
Detection wavelength: 260 nm
Gradient condition: 10% B (0 min) → 85% B (22.5 min) → 100% B (23 min) → 100% B (25 min) → 10% B (26 min) → 10% B (30 min)

### Purification condition 1

Column: Triart Prep C18-S column (10 µm), 10 mm × 250 mm (YMC CO., LTD.)
Mobile phase A: 100 mM aqueous triethylammonium acetate solution
Mobile phase B: acetonitrile
Flow rate: 6.0 mL/min
Column temperature: room temperature
Detection wavelength: 260 nm
Gradient condition: 20 % B (0 min) → 40% B (80 min)

### Desalting condition 1:

Column: Triart Prep C18-S column (10 µm), 10 mm × 250 mm (YMC CO., LTD.)
Mobile phase A: water for injection
Mobile phase B: acetonitrile
Flow rate: 6.0 mL/min
Column temperature: room temperature
Detection wavelength: 260 nm
Gradient condition: 0% B (6.5 min)

### Elution condition 1

Column: Triart Prep C18-S column (10 µm), 10 mm × 250 mm (YMC CO., LTD.)
Mobile phase A: water for injection
Mobile phase B: acetonitrile
Flow rate: 6.0 mL/min
Column temperature: room temperature
Detection wavelength: 260 nm
Gradient condition: 0 % B (0 min) → 45% B (30 min)

### [Example 13-1] Synthesis of PN-S01-10

### 1) Synthesis of a compound with introduced click reagent X

In a glass vial, NS-S01-20 (29.4 mg, 4.0 µmol) was dissolved in a 150 mM phosphate buffer (560 µL, DMSO 68 µL), pH 7.2, to prepare an NS-S01-20 solution. In another glass vial, click reagent X (5.3 mg, 12 µmol) was dissolved in DMSO (690 µL) to prepare a click reagent X solution. 345 µL (1.5 eq) of the click reagent X solution was added to the whole amount of the NS-S01-20 solution and the mixture was stirred at room temperature for 2 hours. Analysis under analysis condition 1 was performed to confirm that the peak of NS-S01-20 decreased to 3% or less, and stirring was stopped.

Retention time (analysis condition 1): NS-S01-20: 9.2 min; click reagent X: 9.5 min

### 2) Synthesis of PN-S01-10

In a glass vial, 13.8 mg of peptide F (6.0 µmol, 1.5 eq.) was dissolved in DMSO (1425 µL) to prepare a peptide F solution. The peptide F solution (1450 µL, 1.5 eq.) was added to the above described solution containing the compound with introduced click reagent X, and the mixture was stirred at room temperature for 2 hours. Analysis under analysis condition 1 was performed to confirm that the peak of the compound with introduced click reagent X decreased to 3% or less, and stirring was stopped. A solution of crude PN-S01-10 with a purity of 64% was obtained.

Retention time (analysis condition 1): a compound with introduced click reagent X: 9.5 min; PN-S01-10: 10.8 min

### [Example 13-2] Purification of PN-S01-10

The solution of crude PN-S01-10 was diluted by adding mobile phase A (118 mL) from purification condition 1, and the diluted solution was used as an injection solution to perform preparative purification under purification condition 1. Fractions were collected over a retention time of 76 minutes to 85 minutes and all fractionated solutions were combined. The fractionated mixture were analyzed under analysis condition 1 to obtain a purified solution of PN-S01-10 with a purity of 90.7% (recovery rate: 100%).

### [Example 13-3] Desalting and elution of PN-S01-10

The purified solution PN-S01-10 was diluted by adding mobile phase A (100 mL) from desalting condition 1, and the diluted solution was used as an injection solution to perform desalting under desalting condition 1. After injecting the injection solution into the column, two column volumes of water for injection were passed through, and the conductivity was measured and confirmed to be 50 µS/cm or less. Then, PN-S01-10 retained in the column was eluted under elution condition 1. Fractions were collected over a retention time of 10 minutes to 13 minutes and all fractionated solutions were combined. The fractionated mixture were analyzed under analysis condition 1 to obtain a desalted solution of PN-S01-10 with a purity of 92.2% (recovery rate: 100%).

### [Example 13-4] Freeze drying of PN-S01-10

The desalted PN-S01-10 solution (42 mL) was diluted by adding water for injection (42 mL). The diluted solution was frozen, and then, dried for 3 days in a freeze-dryer. After drying, PN-S01-10 (16.1 mg, 90.4% pure, 40.4% yield) was obtained as a white solid.

## Claims

1. A compound represented by formula (I): 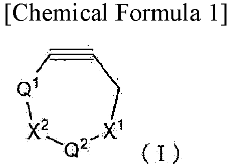 or a salt thereof;
wherein X¹ and X² are each independently an oxygen atom or -N(Y¹)-,
Q¹ is either 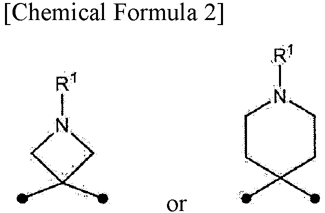 and Q² is selected from C₂₋₄ alkylenes; or
Q¹ is methylene and Q² is either 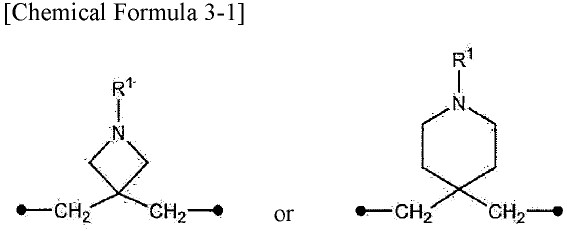 ;
and Y¹ is selected from a hydrogen atom, C₁₋₆ alkanesulfonyl optionally substituted with one or more halogen atoms, and nitrobenzenesulfonyl;
R¹ is selected from a hydrogen atom, C₁₋₆ alkyl, (C₁₋₆ alkyl)carbonyl, (C₁₋₆ alkoxy)carbonyl, benzyloxycarbonyl, [(9H-fluoren-9-yl)methoxy]carbonyl, -CO-Q³-X³, - COO-Q³-X³, -CONR²-Q³-X³, -SO₂-Q³-X³, and -SO₂NR²-Q³-X³;
R² is a hydrogen atom or C₁₋₆ alkyl;
X³ is -COR⁵, -OL¹, -NR³R⁴; or 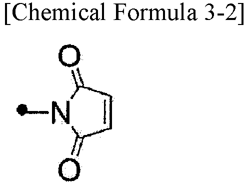
R³ is a hydrogen atom or C₁₋₆ alkyl;
R⁴ is a hydrogen atom, (C₁₋₆ alkyl)carbonyl, (C₁₋₆ alkoxy)carbonyl, -COX⁴, or benzyloxycarbonyl;
Q³ is C₁₋₁₀ alkylene, cyclo C₃₋₁₀ alkylene, C₂₋₁₀ alkylenein which cyclo C₃₋₁₀ alkylene, -(cyclo C₃₋₁₀ alkylene)-(C₁₋₁₀ alkylene)-, -(C₁₋₁₀ alkylene)-(cyclo C₃₋₁₀ alkylene)-, or - (CH₂CH₂O)ₙCH₂CH₂- is inserted;
n is an integer of 1 to 10;
L¹ is a hydrogen atom, -COX⁴, or (C₁₋₆ alkyl)diphenylsilyl;
R⁵ is hydroxy, X⁴, or C₁₋₆ alkoxy; and
X⁴ is 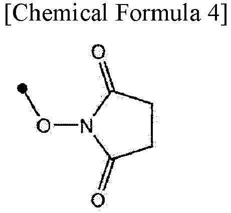 ;
and the triple bond in the compound of formula (I) may be protected by Co₂(CO)₆.

2. The compound according to claim 1 or a salt thereof, wherein Q³ is ethylene or propylene.

3. The compound according to claim 1 or 2 or a salt thereof, wherein R¹ is selected from -CO-CH₂-OH, benzyloxycarbonyl, tert-butoxycarbonyl, and [(9H-fluoren-9-yl)methoxy]carbonyl.

4. The compound according to any one of claims 1 to 3 or a salt thereof, wherein Y¹ is C₁₋₆ alkanesulfonyl, trifluoromethanesulfonyl, and 2-nitrobenzenesulfonyl.

5. The compound according to claim 4 or a salt thereof, wherein Y¹ is methanesulfonyl or tert-butanesulfonyl.

6. The compound according to any one of claims 1 to 5 or a salt thereof, wherein Q¹ is either

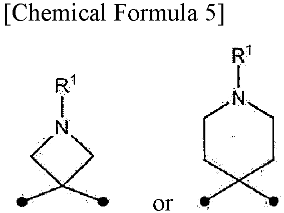

7. The compound according to any one of claims 1 to 5 or a salt thereof, wherein Q² is either

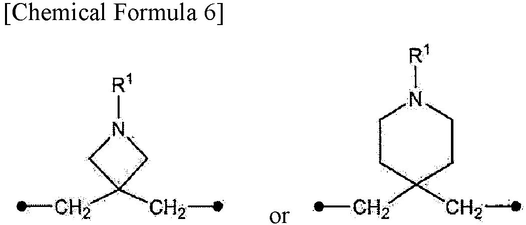

8. The compound according to any one of claims 1 to 5 or a salt thereof,
wherein Q¹ is 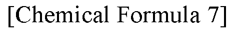 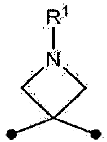 ; and R¹ is benzyloxycarbonyl.

9. The compound according to any one of claims 1 to 5 or a salt thereof,
wherein Q¹ is 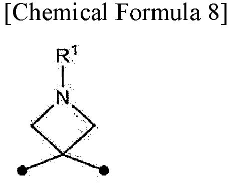 and Q² is selected from C₂₋₃ alkylenes or Q² is 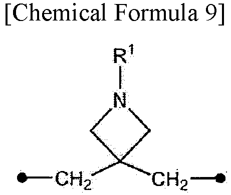 ; and R¹ is benzyloxycarbonyl.

10. The compound according to claim 1 or a salt thereof, wherein the compound is selected from:

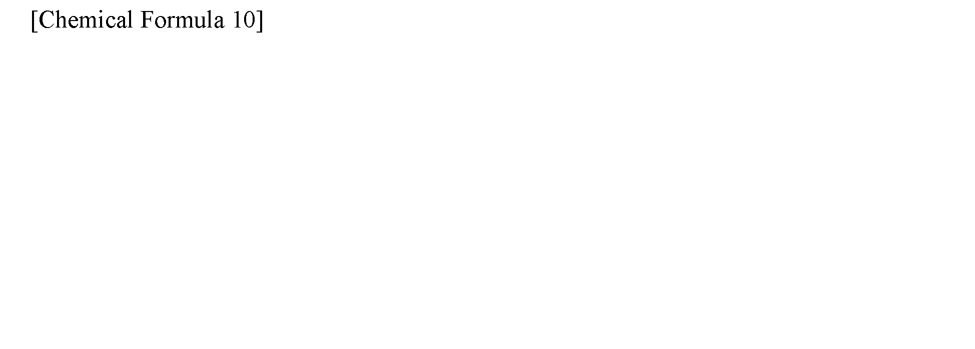

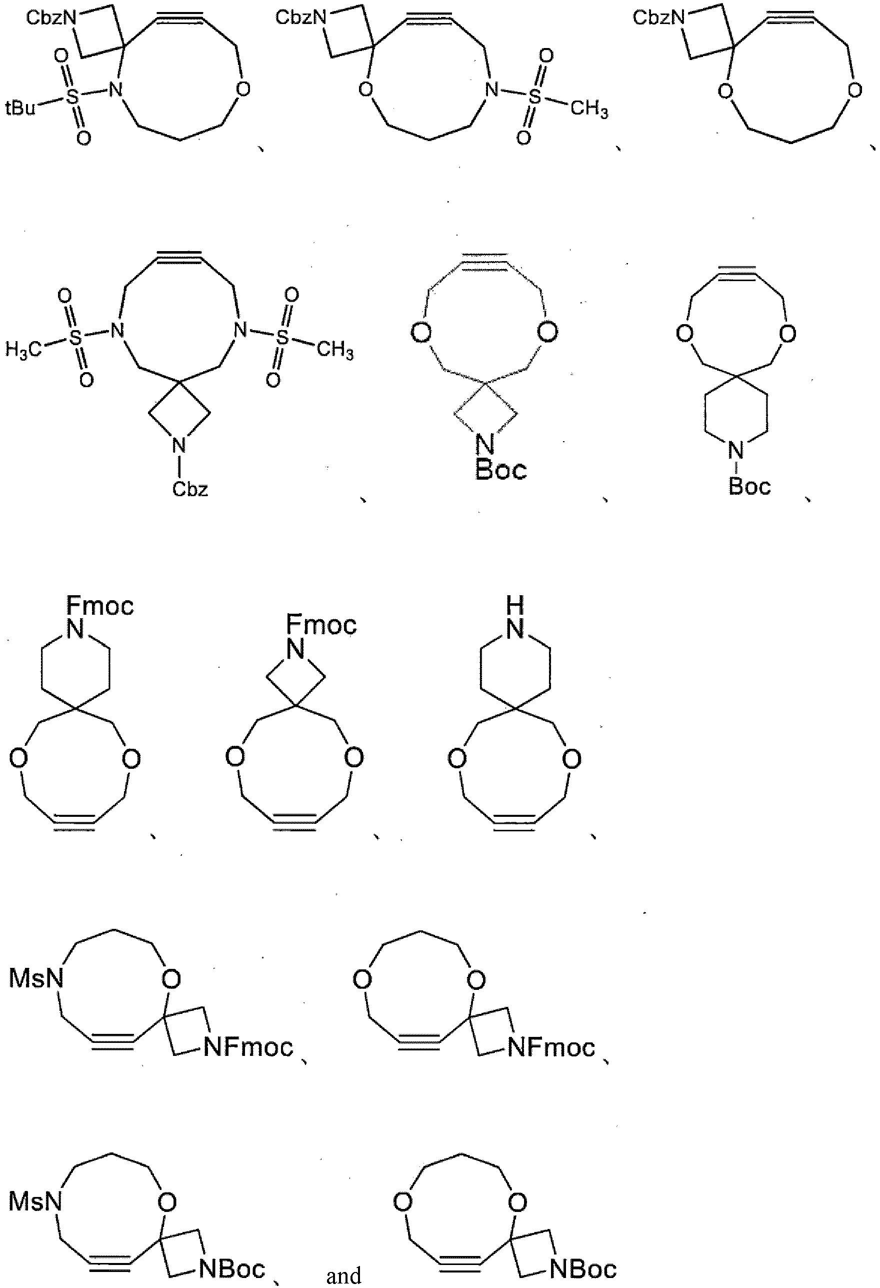

11. A method for producing a conjugate of functional molecules by using the compound according to any one of claims 1 to 10 or a salt thereof, the method comprising:
reacting a first functional molecule having a group represented by formula (II): 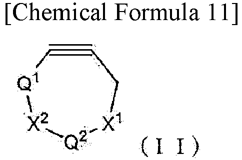
wherein X¹ and X² are each independently an oxygen atom or -N(Y¹)-,
Q¹ is either 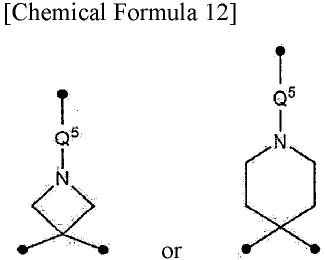 and Q² is selected from C₂₋₄ alkylenes; or
Q¹ is methylene and Q² is either 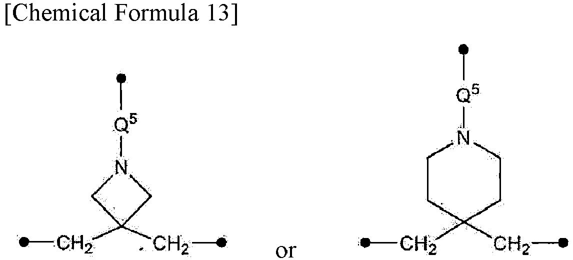 ;
Q⁵ is a linker or a single bond attached to the first functional molecule with a second functional molecule having a group represented by formula (III):
-Q⁶-N₃ (III)
wherein Q⁶ is a linker or a single bond attached to the second functional molecule to obtain a conjugate comprising the first functional molecule and the second functional molecule linked by a linker, represented by formula IVa or formula IVb:

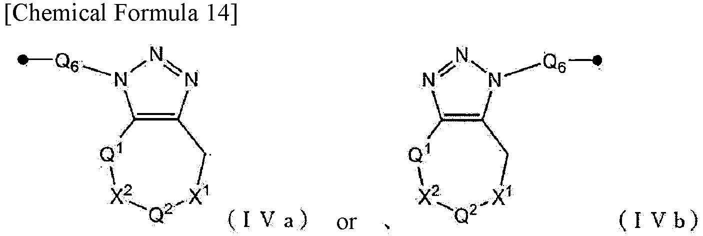

12. The method according to claim 11, wherein the first functional molecule and the second functional molecule are each independently a pharmacologically active compound, a label compound, a peptide, a protein, a nucleic acid, or a molecule used for a drug delivery system.

13. The method according to claim 12, wherein either the first functional molecule or the second functional molecule is a pharmacologically active compound, a label compound, a peptide, a protein, or a nucleic acid that has a specific binding activity to a target.

14. A conjugate comprising a first functional molecule and a second functional molecule linked by a linker, represented by formula (IVa) or formula (IVb): 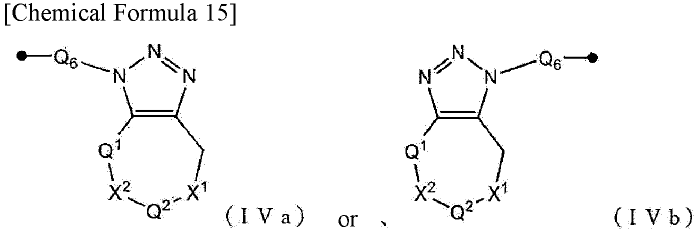 wherein X¹, X², Q¹, Q², and Q⁶ are as defined in claim 11.

15. The conjugate according to claim 14, wherein the first functional molecule and the second functional molecule are each independently a pharmacologically active compound, a label compound, a peptide, a protein, a nucleic acid, or a molecule used for a drug delivery system.

16. The conjugate according to claim 15, wherein either the first functional molecule or the second functional molecule is a pharmacologically active compound, a label compound, a peptide, a protein, or a nucleic acid that has a specific binding activity to a target.

17. A method for producing a compound represented by formula (I): 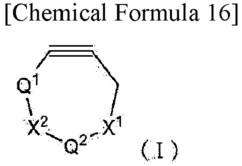 wherein X¹, X², Q¹, and Q² are as defined in claim 1, and the triple bond in the compound of formula (I) may be protected by Co₂(CO)₆;
the method comprising:
treating a compound represented by formula (V): 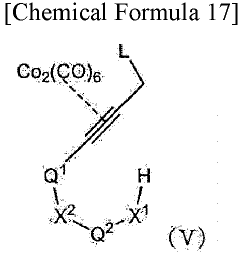
wherein X¹, X², Q¹, and Q² are as defined in claim 1, with the provision that any hydroxy group and amino group, if present, is protected by a protecting group; and L is a leaving group,
in the presence of an acid to obtain a corresponding cyclic compound.

18. The method according to claim 17, wherein the acid is a Lewis acid or a Bronsted acid.

19. The method according to claim 17, wherein L is -OR¹⁰ or a halogen atom; and R¹⁰ is a hydrogen atom, (C₁₋₆ alkyl) carbonyl, acetyl, tri(C₁₋₆ alkyl)silyl, C₁₋₆ alkyl, benzenesulfonyl, or C₁₋₆ alkanesulfonyl.
